# EUROPEAN PATENT SPECIFICATION

(11) **EP 4 499 865 B1**
(45) Date of publication and mention of the grant of the patent: **10.12.2025**
(21) Application number: 23709022.0
(22) Date of filing: 13.02.2023
(51) Int. Cl.: C12Q 1/6834

(54) **AUTHENTICATION ASSAY USING EMBEDDED DEOXYRIBONUCLEIC ACID TAGGANTS**
AUTHENTIFIZIERUNGSTEST UNTER VERWENDUNG EINGEBETTETER DESOXYRIBONUKLEINSÄURE-TAGGERANTEN
DOSAGE D'AUTHENTIFICATION UTILISANT DES TRACEURS D'ACIDE DÉSOXYRIBONUCLÉIQUE INCORPORÉS

(30) Priority: 29.03.2022 US 202217657120
(43) Date of publication of application: 05.02.2025
(73) Proprietor: Microsoft Technology Licensing, LLC, Redmond, WA 98052-6399 (US)
(72) Inventor: CHEN, Yuan-Jyue, Redmond, Washington 98052-6399 (US); STRAUSS, Karin, Redmond, Washington 98052-6399 (US); CHANDRA, Ranveer, Redmond, Washington 98052-6399 (US)
(74) Representative: Goddar, Heinz J.
(86) International application number: PCT/US2023/012878
(87) International publication number: WO 2023/191961

(56) References cited:
- WO-A1-2019/183359
- WO-A2-2018/057502
- BERK KIMBERLY L. ET AL: "Rapid Visual Authentication Based on DNA Strand Displacement", APPLIED MATERIALS & INTERFACES, vol. 13, no. 16, 28 April 2021 (2021-04-28), US, pages 19476 - 19486, XP055858211, ISSN: 1944-8244, Retrieved from the Internet <URL:https://pubs.acs.org/doi/pdf/10.1021/acsami.1c02429> DOI: 10.1021/acsami.1c02429

## Description

### BACKGROUND

Due to the ease by which rare, expensive, and/or brand-name products may be forged, the likelihood of consumers inadvertently purchasing counterfeit products is on the rise. To prevent fraud and the economic impact thereof in these industries, high-value commercial products, works of art, currency, wine and spirits, and the like often include indications of authenticity. These anti-counterfeiting technologies provide evidence for the consumer that the product is genuine, and are important for economic stability of the markets impacted by forgery and fraud.

BERK KIMBERLY L. ET AL: "Rapid Visual Authentication Based on DNA Strand Displacement", APPLIED MATERIALS & INTERFACES, vol. 13, no. 16, 28 April 2021 (2021-04-28), pages 19476-19486, XP055858211, describes novel ways to track and verify items of a high value or security. Taggants made from deoxyribonucleic acid (DNA) have several advantageous properties, such as high information density and robust synthesis; however, existing methods require laboratory techniques to verify, limiting applications. Here, we leverage DNA nanotechnology to create DNA taggants that can be validated in the field in seconds to minutes with a simple equipment. The system is driven by toehold-mediated strand-displacement reactions where matching oligonucleotide sequences drive the generation of a fluorescent signal through the potential energy of base pairing. By pooling different "input" oligonucleotide sequences in a taggant and spatially separating "reporter" oligonucleotide sequences on a paper ticket, unique, sequence-driven patterns emerge for different taggant formulations. Algorithmically generated oligonucleotide sequences show no crosstalk and ink-embedded taggants maintain activity for at least 99 days at 60°C (equivalent to nearly 2 years at room temperature). The resulting fluorescent signals can be analyzed by the eye or a smartphone when paired with a UV flashlight and filtered glasses.

### SUMMARY

To address the issues discussed herein, an authentication assay using embedded deoxyribonucleic acid (DNA) taggants is provided. According to one aspect, the authentication assay may include a substrate and a sample of an authenticity label collected from a product. The substrate may have a plurality of physically separated assay locations. Each assay location of the plurality of assay locations may include a reporter oligonucleotide bound to the substrate via an anchor region. Each reporter oligonucleotide bound to each assay location of the plurality of assay locations may include a first region, a second region, and a third region. The first region may include a single-stranded toehold sequence that is common among each of the reporter oligonucleotides bound to each assay location of the plurality of assay locations. The second region may include a universal sequence that is common among each of the reporter oligonucleotides bound to each assay location of the plurality of assay locations. The third region may include unique sequence that is different for each of the reporter oligonucleotides bound to each assay location of the plurality of assay locations. The second and third regions of each reporter oligonucleotide may be prehybridized with a complementary strand having a sequence that is complementary to the second and third regions. The sample may include at least one DNA taggant having a sequence complementary to the first and second regions of each of the reporter oligonucleotides, and complementary to the third region of at least one reporter oligonucleotide bound to an assay location of the plurality of assay locations. The at least one DNA taggant may include a fluorophore molecule configured to emit light upon excitation. Incubation of the substrate with the sample may result in binding of the at least one DNA taggant to the at least one reporter oligonucleotide, thereby initiating a toehold-mediated DNA strand displacement reaction that exchanges the complementary strand of the at least one reporter oligonucleotide for the at least one DNA taggant including the fluorophore molecule. Excitation of the fluorophore molecule attached to the DNA taggant may produce a pattern of emitted light at one or more assay locations.

According to another aspect, a method for manufacturing an authentication assay using DNA taggants is provided. The method may include preparing a substrate with a plurality of physically separated assay locations. The method may further include synthesizing a plurality of reporter oligonucleotides, each of which has a first region including a single-stranded toehold sequence that is common among each of the reporter oligonucleotides, a second region including a universal sequence that is common among each of the reporter oligonucleotides, and a third region including a unique sequence that is different for each of the reporter oligonucleotides. The method may further include prehybridizing the second and third regions of each reporter oligonucleotide of the plurality of reporter oligonucleotides with a complementary strand having a sequence that is complementary to the second and third regions of each reporter oligonucleotide. The method may further include binding, via an anchor region, each reporter oligonucleotide of the plurality of reporter oligonucleotides to one of the plurality of physically separated assay locations such that each assay location includes a reporter oligonucleotide with a unique third region. The method may further include synthesizing at least one DNA taggant to have a sequence complementary to the first and second regions of each of the reporter oligonucleotides, and complementary to the third region of at least one reporter oligonucleotide bound to an assay location of the plurality of assay locations, the at least one DNA taggant including a fluorophore molecule configured to emit light upon excitation. The method may further include adding the at least one DNA taggant to an authenticity label configured to verify an authenticity of a product.

Incubation of the substrate with the sample may result in binding of the at least one DNA taggant to the at least one reporter oligonucleotide, thereby initiating a toehold-mediated DNA strand displacement reaction that exchanges the complementary strand of the at least one reporter oligonucleotide for the at least one DNA taggant including the fluorophore molecule. Excitation of the fluorophore molecule attached to the DNA taggant may produce a pattern of emitted light at one or more assay locations.

According to another aspect, an authentication assay using embedded DNA taggants is provided. The authentication assay may include a substrate and a sample of an authenticity label collected from a product. The substrate may have a plurality of physically separated assay locations. Each assay location of the plurality of assay locations may include a reporter oligonucleotide bound to the substrate via an anchor region. Each reporter oligonucleotide bound to each assay location of the plurality of assay locations may include a first region and a second region. The first region of each reporter oligonucleotide may include a universal sequence that is common among each of the reporter oligonucleotides bound to each assay location of the plurality of assay locations. The second region of each reporter oligonucleotide includes a unique sequence that is different for each of the reporter oligonucleotides bound to each assay location of the plurality of assay locations. The first and second regions of each reporter oligonucleotide may be prehybridized with a complementary strand having a sequence that is complementary to the first and second regions of each reporter oligonucleotide. The complementary strand may include a toehold sequence and a fluorophore molecule. The sample may include at least one DNA taggant having a same sequence as the first and second regions of at least one reporter oligonucleotide bound to an assay location of the plurality of assay locations, and a toehold sequence complementary to the toehold sequence of the complementary strand. Incubation of the substrate with the sample may result in binding of the at least one DNA taggant to the complementary strand, thereby initiating a toehold-mediated DNA strand displacement reaction that displaces the complementary strand, including the fluorophore molecule, from the at least one reporter oligonucleotide. Exposure of the substrate to light configured to excite fluorophore molecules may produce a pattern of emitted light at one or more assay locations, the pattern including an absence of emitted light at one or more assay locations of the plurality of assay locations due to release of the complementary strand, including the fluorophore molecule.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not limited to implementations that solve any or all disadvantages noted in any part of this disclosure.

### BRIEF DESCRIPTION OF THE DRAWINGS

FIGS. 1A to 1C are illustrations showing DNA strand displacement in an authentication assay using embedded DNA taggants, according to a first embodiment of the disclosure.
FIG. 2 is a diagram showing replacement of a DNA strand complementary to a reporter oligonucleotide with a fluorophore-labeled DNA taggant of the authentication assay of FIGS. 1A to 1C.
FIG. 3 is an illustration of physically separated assay locations on a substrate of the authentication assay of FIGS. 1A to 1C.
FIGS. 4A and 4B are diagrams of the DNA taggant and a decoy DNA taggant, respectively, of the authentication assay of FIGS. 1A to 1C.
FIG. 5 is an illustration showing addition of an authenticity label of the authentication assay of FIGS. 1A to 1C to a product.
FIG. 6 is an illustration showing results of the authentication assay of FIGS. 1A to 1C.
FIG. 7 is an illustration showing an example of verifying results of the authentication assay of FIGS. 1A to 1C.
FIGS. 8A to 8C are illustrations showing DNA strand displacement in an authentication assay using embedded DNA taggants, according to a second embodiment of the disclosure.
FIG. 9 is a diagram showing displacement of a fluorophore-labeled DNA strand complementary to a reporter oligonucleotide with a DNA taggant of the authentication assay of FIGS. 8A to 8C.
FIG. 10 is an illustration of physically separated assay locations on a substrate of the authentication assay of FIGS. 8A to 8C.
FIGS. 11A and 11B are diagrams of the DNA taggant and a DNA decoy taggant, respectively, of the authentication assay of FIGS. 8A to 8C.
FIG. 12 is an illustration showing addition of an authenticity label of the authentication assay of FIGS. 8A to 8C to a product.
FIG. 13 is an illustration showing results of the authentication assay of FIGS. 8A to 8C.
FIG. 14 is an illustration showing an example of verifying results of the authentication assay of FIGS. 8A to 8C.
FIGS. 15A and 15B are illustrations showing how the DNA taggants of the authentication assays of FIGS. 1A to 1C and 8A to 8C, respectively, are unable to be mimicked via polymerase chain reaction (PCR).
FIG. 16 is a flowchart of a method for manufacturing a multiplex assay for nucleic acid detection according to one example configuration of the present disclosure.

### DETAILED DESCRIPTION

Several significant challenges exist in distinguishing counterfeit goods from authentic goods. Anti-counterfeit technologies, such as holograms, optical strips, radio frequency identification (RFID), quick response (QR) codes, and near-field communication (NFC) add security to a product and provide evidence that the product is genuine. However, these technologies may be lost or damaged after application, difficult to attach to products, and easy to copy. Additionally, it may not be feasible to use such tagging mechanisms with products that are small, flexible, or numerous in quantity, or in scenarios in which the code should be invisible to the naked eye.

An assay using embedded molecular tags would provide an anti-counterfeit technology that is difficult to mimic. Molecular tagging is inexpensive, fast, reliable, simple to decode, and would thus improve the efficiency of conducting authentication analyses and negate the need for performing several individual assays. Further, in combination with the above features, the ability to detect results of the assay quickly and easily with a hand-held light and mobile computing device would enable a user to perform the assay from start to finish in virtually any location. An assay having these characteristics would offer the ability to confirm authenticity of a product quickly and reliably without the requirement for large equipment and laboratory space. However, heretofore challenges have existed for the development of such an assay.

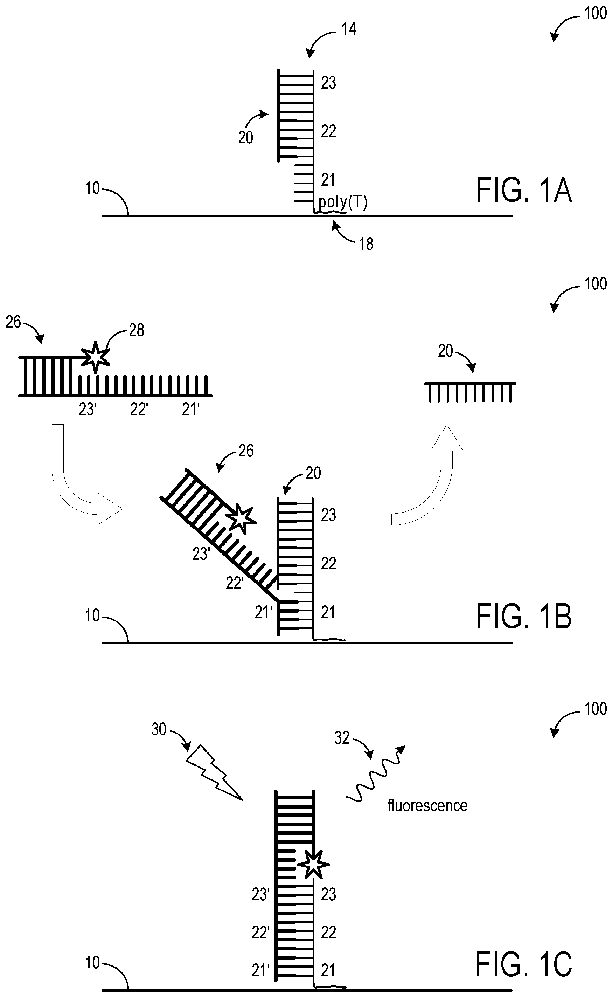

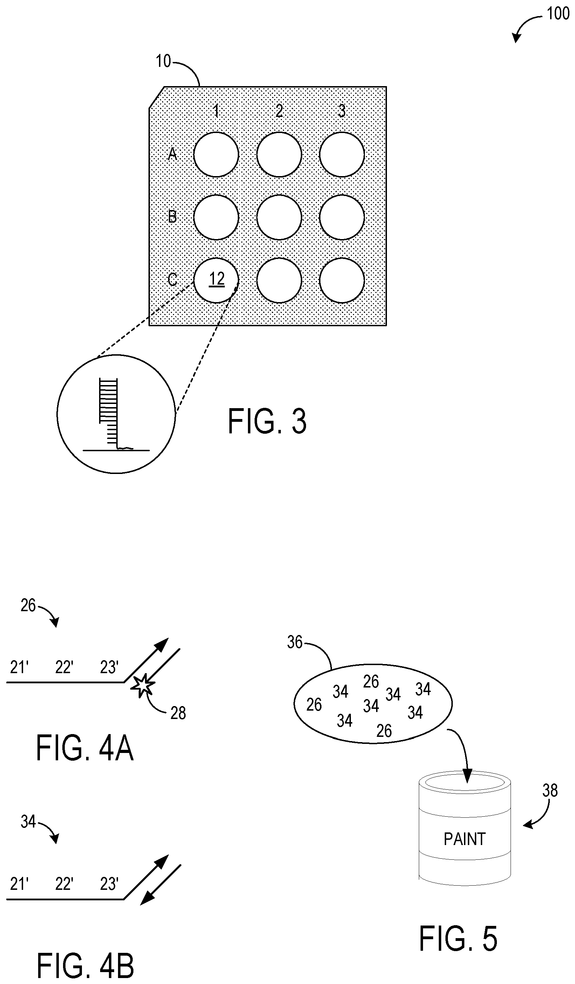

As illustrated in FIG. 1A, to address the above identified issues, a first embodiment of an authentication assay 100 using embedded DNA taggants is provided. The authentication assay 100 includes a substrate 10. As described below with reference to FIG. 3, the substrate 10 has a plurality of physically separated assay locations 12. Each assay location 12 of the plurality of assay locations includes a reporter oligonucleotide 14. The reporter oligonucleotide 14 includes an anchor region 18, a first region 21, a second region 22, and a third region 23. The anchor region 18 binds the reporter oligonucleotide 14 to the substrate 10. The first region 21 is a single-stranded region that is a toehold sequence for a toehold-mediated strand displacement reaction. The second region 22 of the reporter oligonucleotide 14 is a universal sequence that is common among the reporter oligonucleotides 14 at each of the plurality of physically separated assay locations 12. The third region 23 is a unique sequence that is different for each reporter oligonucleotide 14. The second and third regions 22, 23 are prehybridized with a complementary strand 20 having a sequence that is complementary to the second and third regions 22, 23 of the reporter oligonucleotide 14. It will be appreciated that the reporter oligonucleotide 14 is preferably at least 15 nucleotides long, and more preferably 20 nucleotides to 30 nucleotides long, with the toehold region comprising 8 or fewer nucleotides.

The reporter oligonucleotide 14 is bound to the substrate 10 via the anchor region 18. The substrate 10 may be a nitrocellulose membrane, as it is widely used for immobilizing nucleic acids. The anchor region 18 may be designed to be a poly-thymine ((poly)T) tail at the 3' end of the reporter oligonucleotide 14 that binds to the nitrocellulose membrane. However, it will be appreciated that the (poly)T tail may be located at the 5' end of the reporter oligonucleotide, and/or the reporter oligonucleotide 14 may form a stable interaction with the nitrocellulose membrane via a different mechanism, such as modifying an end of the reporter oligonucleotide 14 with biotin such that it can form a strong bond with streptavidin-coated nitrocellulose membrane, for example. Additionally or alternatively, the substrate may be an eggshell membrane (ESM), for example. It has been demonstrated that ESM treated with acetic acid or n-butyl acetate can be used to immobilize nucleic acids having terminal amine groups. Other substrates, such as nylon membranes, nanofibers, chitosan-modified membranes, for example, may be used to bind and retain nucleic acids in the multiplex assay described herein.

The authentication assay 100 further includes a sample 24 of an authenticity label 36 collected from a product 38, as described in detail below with reference to FIG. 5. The sample 24 may be collected by swabbing a solid material or extracting a portion of a liquid product, for example. The sample 24 includes at least one DNA taggant 26, as illustrated in FIG. 1B. The DNA taggant 26 has first, second, and third regions 21', 22', and 23', respectively, that are complementary to the first (i.e., toehold), second, and third regions 21, 22, 23 of the reporter oligonucleotide. The DNA taggant 26 additionally includes a fluorophore molecule 28. The sample 24, including the DNA taggant 26, may be added to a liquid reagent to be used as an incubation solution for the substrate 10. The liquid reagent may be contained in a vessel such as a zip-top bag or a leak-proof container, for example, that is configured for incubation of the substrate 10 with the sample 24 such that each assay location 12 is in contact with the sample 24, including the DNA taggant 26. During incubation, the DNA taggant 26 binds to the toehold region 21 of the reporter oligonucleotide 14 via the first region 21' that is complementary to the toehold region 21, as illustrated in FIG. 1B. This binding initiates a toehold-mediated DNA strand displacement reaction in which the complementary strand 20 bound to the second and third regions region 22, 23 of the reporter oligonucleotide 14 is exchanged for the second and third regions 22', 23' of the DNA taggant 26. The complementary strand 20 is released and removed from the assay when the sample 24 is washed from the substrate 10 with a neutral buffer. It will be appreciated that the substrate 10 including the bound reporter oligonucleotide 14, authenticity label 36, liquid reagent, and wash buffer may be stably stored and used at room temperature, and no special equipment is required for the toehold-mediated DNA strand displacement reaction.

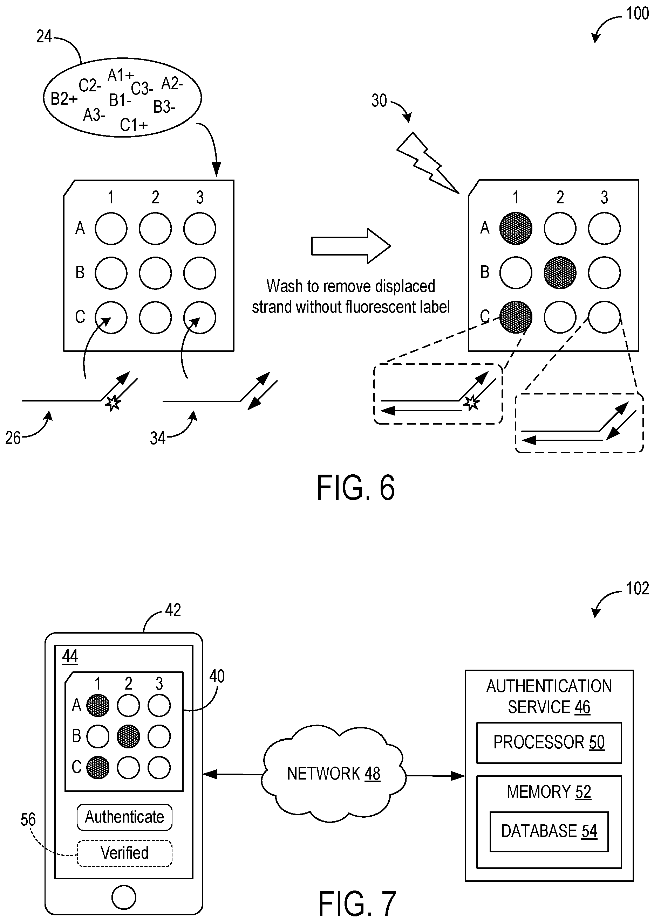

The fluorophore molecule 28 is attached to the DNA taggant 26, and is thus configured to label the reporter oligonucleotide 14 when the toehold-mediated DNA strand displacement reaction results in binding of the second and third regions 22', 23' of the DNA taggant 26 to the first, second, and third regions region 21, 22, 23 of the reporter oligonucleotide 14. As shown in FIG. 1C, when the fluorophore molecule 28 is excited by exposure to light 30, its fluorescence can be detected as an emission of light 32 at a predetermined wavelength. It will be appreciated that light described herein to which the fluorophore molecule 28 is exposed may be ultraviolet (UV) light, light from a light-emitting diode (LED), or another type of light emitting device suitable for excitation of the fluorescent dye. It is preferable that the light emitting device be portable, and it is also preferable that the light source be configured as a handheld light. Exposure of the substrate 10 to light produces a pattern of emitted light 32 at one or more of the plurality of assay locations due to excitation of the fluorophore molecule 28 attached to the DNA taggant 26. As described in detail below with reference FIGS. 6 and 7, a user can verify an authenticity of the product via the pattern.

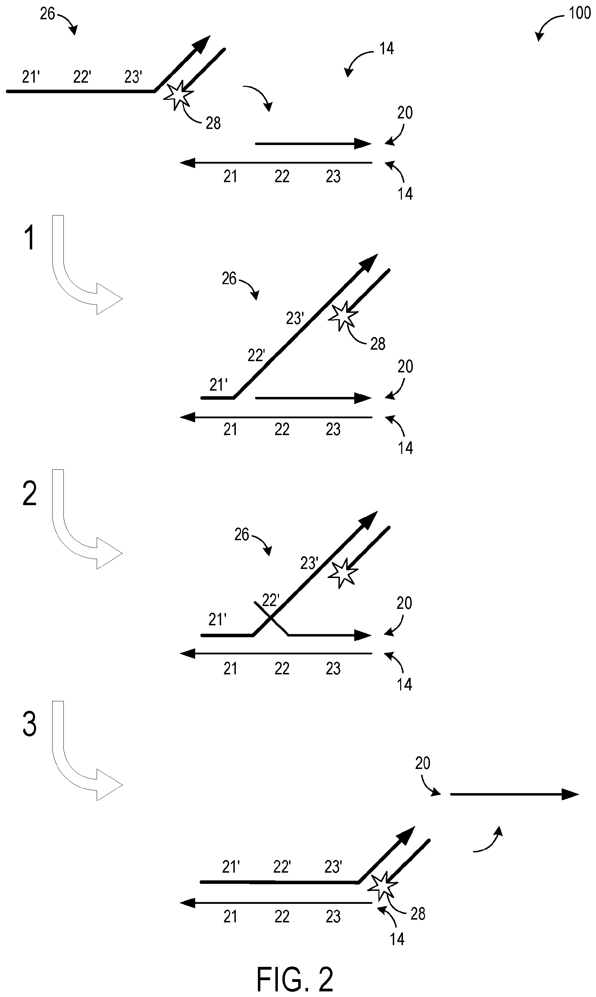

FIG. 2 illustrates a diagram of the replacement of the complementary strand 20 with the fluorophore-labeled DNA taggant 26. It will be appreciated that the DNA strands are shown to have directionality from the 5' to the 3' end, as is conventional. As described above and shown in FIG. 2, when the DNA taggant 24 contacts the assay location 12 that includes the reporter oligonucleotide 14, the first region 21' at the 5' end of the DNA taggant 26, which is complementary to the 3' toehold region 21 of the reporter oligonucleotide, binds to the toehold region 21 (indicated by arrow 1). This binding initiates a toehold-mediated DNA strand displacement reaction, in which the 5' end of the complementary strand 20 is exchanged for the second and third regions 22', 23' of the DNA taggant 26 (indicated by arrow 2). The complementary strand 20 is released and removed from the authentication assay 100 (indicated by arrow 3).

FIG. 3 shows an example of physically separated assay locations 12 on the substrate 10 of the authentication assay 100. As illustrated, a notch or the like may be formed in a corner of the substrate 10 for the purpose of orientation. In the example shown in FIG. 3, the authentication assay 100 is configured as a spot assay in which each assay location 12 is defined as an individual spot on the substrate 10. However, it will be appreciated that the authentication assay 100 may be configured as a lateral flow assay, for example.

Each assay location 12 of the plurality of assay locations may have a reporter oligonucleotide 14 with a unique sequence. An example reporter oligonucleotide 14 is depicted in the enlarged illustration of the assay location 12 in FIG. 3. To ensure that similar quantities of the reporter oligonucleotides 14 are precisely and reproducibly added to each assay location 12, the reporter oligonucleotides 14 may be deposited, i.e., printed, on the substrate 10 using an inkjet printer or the like. Alternatively, the reporter oligonucleotides may be deposited using a micropipette or another suitable instrument capable of accurately transferring precise quantities of the components. When the authentication assay 100 is ready to be used, it can simply be submerged in a volume of liquid reagent containing the sample 24.

As described above and shown in the figures, the plurality of assay locations 12 on the substrate 10 are physically separated from one another. In one implementation, the physical separation of the assay locations 12 is achieved via depositing a pattern of barriers on the substrate. Specifically, when the substrate 10 is configured as a nitrocellulose membrane, a wax-based printer may be used to deposit wax to form the pattern of barriers. When the wax is heated, it permeates the nitrocellulose membrane, thereby forming hydrophobic barriers to physically separate the assay locations 12 from one another, as depicted by the shaded portions of FIG. 3. While the example authentication assay 100 illustrated in FIG. 3 has nine assay locations 12, it will be appreciated that the authentication assay 100 may be configured to have any suitable number of assay locations 12. It will be further appreciated that forming hydrophobic barriers from wax is merely one method of physically separating the assay locations 12, and is not meant to limit the scope of how the physical separation is achieved.

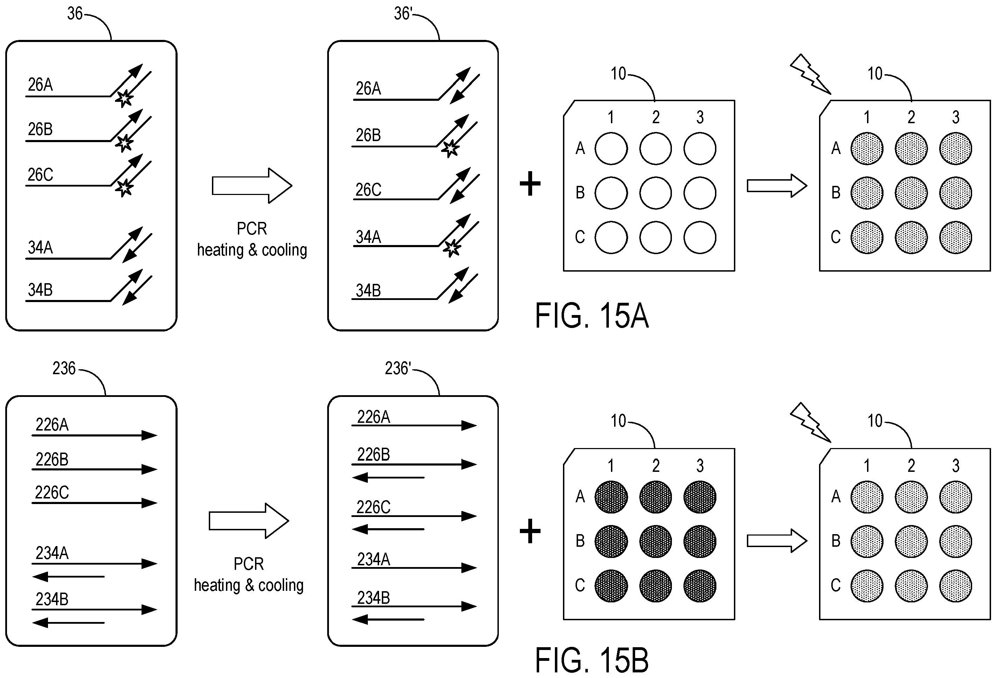

FIGS. 4A and 4B are diagrams of the DNA taggant 26 and a decoy DNA taggant 34, respectively, of the authentication assay 100. As described above, the DNA taggant 26 includes a fluorophore molecule 28, which labels the reporter oligonucleotide 14 that is bound to the substrate 10 as a result of the toehold-mediated DNA strand displacement reaction. The decoy DNA taggant 34 is configured similarly to the DNA taggant 26, but lacks the fluorophore molecule 28. As such, when the decoy DNA taggant 34 binds to the reporter oligonucleotide 14, the reporter oligonucleotide 14 is not labeled with the fluorophore molecule 28. As described in detail below with reference to FIG. 6, the combination of DNA taggants 26 and decoy DNA taggants 34 in the sample 24 of the authenticity label 36, and the reporter oligonucleotides 14 in the assay locations 12 to which the DNA taggants 26 and decoy DNA taggants 34 are complimentary, form a fluorescent authenticity pattern on the substrate 10 upon excitation of the fluorophore molecules 28. In some implementations, the decoy DNA taggant 34 may include the fluorophore molecule 28, but have a different sequence at the third region, thereby blocking the toehold-mediated DNA strand displacement reaction. Additionally, as discussed below with reference to FIGS. 15A and 15B, the combination of DNA taggants 26 and decoy DNA taggants 34 in the authenticity label 36 make it difficult to mimic the DNA taggants 26 and reproduce the fluorescent authenticity pattern on the substrate 10, thereby protecting the authenticity labels 36 against counterfeiting and forgery.

The addition of the authenticity label 36 to a product 38 is illustrated in FIG. 5. The product 38 may be a commercial product, such as a high-value item, currency, food and agricultural products, goods likely to be forged, or the like. As described below with reference to FIG. 6, the assay 100 can be configured to store binary data. As such, while the authenticity label 36 is configured to verify authentic products and identify counterfeit products, additional information may be encoded in the sample via the DNA taggants 26 and decoy DNA taggants 26 and the resulting pattern of fluorescence on the substrate 10, such as a source of the product, materials used in the manufacture of the product, and any processes the product has undergone, for example. The authenticity label 36 may be added directly to liquid products, powdered products, and/or applied to solid products.

In the example shown in FIG. 5, the authenticity label 36 is added to a liquid product 38, specifically paint. The authentication assay 100 may be configured to display a pattern based on the paint, a work of art, or a particular artist, for example. As such, a user could swab the painting to remove the sample 24 of the authenticity label 36 to verify the authenticity of a high-value painting via the authentication assay 100. Authentication labels can be added to other liquids, such as fine wine or rare spirits, for example, which would a user to verify their purchase and dissuade forgery or re-filling of luxury bottles with inexpensive spirits, thereby protecting the economy of the industry.

FIG. 6 illustrates analyzing the sample 24 with the authentication assay 100, and the resulting pattern of the authentication assay 100 upon excitation of the fluorophore molecules 28. As described above, the sample 24 of the authenticity label 36 may be swabbed from the product 38 and placed in an amount of liquid reagent to distribute the DNA taggants 26 throughout the sample 24, thereby enabling preparation of the sample 24 for use with the authentication assay 100 at any number of locations.

In the example shown in FIG. 6, the sample 24 is depicted as containing three DNA taggants 26 and six decoy DNA taggants. However, it will be appreciated that any suitable number of DNA taggants 26 and decoy DNA taggants 34 may be included in the authenticity label 36. For the sake of simplicity, the DNA taggants contained in the sample 24 of the authenticity label 36 are indicated by "A1+," "B2+," and "C1+," and the decoy DNA taggants are indicated by "A2-," "A3-," "B1-," "B3-," "C2-," and "C3-," corresponding to the indicated assay locations 12. As an example, when the DNA taggant 26 binds to the reporter oligonucleotide 14 in assay location C1, that assay location fluoresces upon excitation of the fluorescent molecule 28 on the DNA taggant 26, as indicated by the shading in assay location C1. In contrast, when the decoy DNA taggant 34 binds to the reporter oligonucleotide 14 in assay location C3, no fluorescence is observed. As such, the substrate 10 displays a fluorescent authenticity pattern 40 after incubation of the substrate 10 with the sample 24.

As indicated above, the authenticity label 36 may be configured to encode binary data. For example, the DNA strands of the taggants that include the first, second, and third regions 21', 22', 23' may be defined as DNAbits. In the assay 100 described herein, the fluorophore-labeled DNA taggants 26 may encode 1s of DNAbits, and the decoy DNA taggants 34 that lack the fluorophore molecule 28 may encode 0s of DNAbits. The binding of the DNA taggants 26 and decoy DNA taggants 34 to reporter oligonucleotides 14 bound to different assay locations 12 result conversion of the 1s and 0s encoded in the DNAbits to a pattern of fluorescence, thereby enabling the authenticity of a product, as well as additional data, to be determined in a single, multiplex assay that simplifies the reading process.

A system 102 for analyzing the authentication assay 100 may include a light emitting device, an optical reader 42, and a computing device 46. The light emitting device may be a handheld light, as described above with reference to FIG. 1C, configured to emit light 30 at a wavelength that causes excitation of the fluorophore molecule 28 attached to the DNA taggants 26. The optical reader 42 is configured to capture the pattern of emitted light 30, and may be implemented as a camera-equipped mobile computing device. The computing device 46 is configured to receive data encoding the pattern of emitted light 30 from the optical reader 42, perform a verification action on the pattern of emitted light 30, and output a verification result. The computing device 46 may be implemented as a server configured to receive the data encoding the pattern of emitted light 30 over a computer network 48 from the mobile computing device 42.

An example of the system 102 for analyzing the authentication assay 100 is shown in FIG. 7. To verify the authenticity label 36 or read the binary data based on the fluorescent authenticity pattern 40, the user may capture an image of the fluorescent authenticity pattern 40 with a cellular phone 42. Using a verification application with a user interface 44, the data encoding the fluorescent authenticity pattern 40 may be transmitted to an authentication server 46 via a cloud-based network 48. The authentication server 46 includes a processor 50 and memory 52 storing a database 54 of authenticity patterns. The fluorescent authenticity pattern 40 is analyzed and compared against a fluorescent authenticity pattern database 54. If the fluorescent authenticity pattern 40 matches the correct pattern in the fluorescent authenticity pattern database 54, a verification message 56 is transmitted to the user and displayed on the cellular phone 42 via the user interface 44. Additionally or alternatively, it will be appreciated that the authenticity label 36 may be verified by the user by comparing the fluorescent authenticity pattern 40 to a pattern key. For example, a product for purchase may come with a printed pattern key, or have a virtual pattern key available on an associated website, to allow the user to verify the authenticity of the product without transmitting the fluorescent authenticity pattern 40 to the authentication server 46, for example.

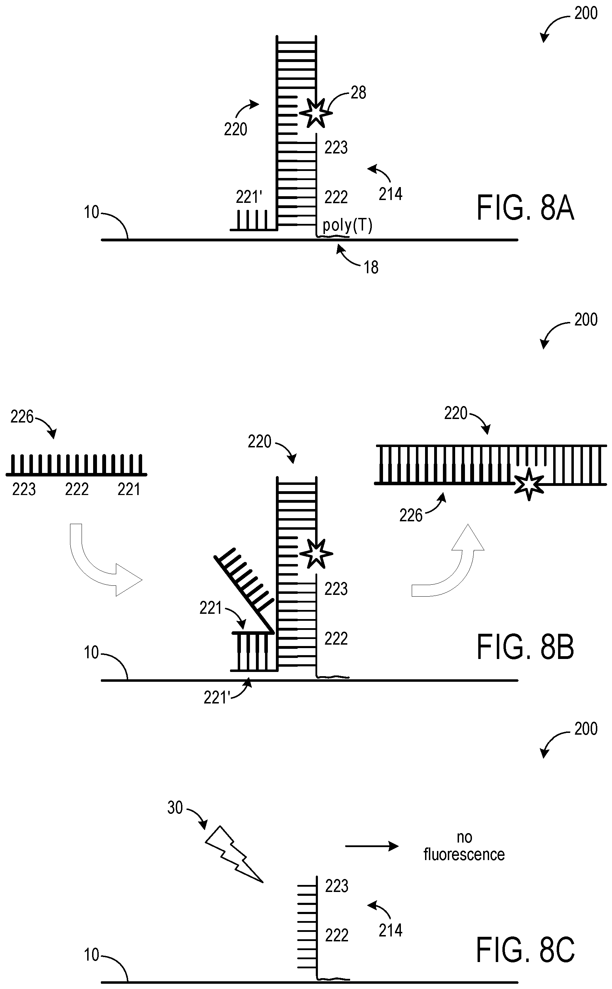

A second embodiment of an authentication assay 200 using embedded DNA taggants will now be described. FIGS. 8A to 8C illustrate the authentication assay 200 using embedded DNA taggants. Like the authentication assay 100, the authentication assay 200 includes the substrate 10 with a plurality of physically separated assay locations 212, each of which includes a reporter oligonucleotide 214. However, in the authentication assay 200, the reporter oligonucleotide 214 is prehybridized with a complementary strand 220 that includes the fluorophore molecule 28, which is in contrast the configuration of the authentication assay 100 in which the DNA taggant 26 is labeled with the fluorophore molecule 28.

As shown in FIG. 8A, the reporter oligonucleotide 214 is bound to the substrate 10 via the anchor region 18. As with the authentication assay 100, the substrate 10 may be a nitrocellulose membrane, and the anchor region 18 may be a poly-thymine ((poly)T) tail at the 3' or 5' end of the reporter oligonucleotide 214 that binds to the nitrocellulose membrane. A first region 222 of the reporter oligonucleotide 214 is a universal sequence that is common among the reporter oligonucleotides 214 at each of the plurality of physically separated assay locations 212. A second region 223 has a unique sequence that is different for each reporter oligonucleotide 214. The second and third regions 222, 223 are prehybridized with the complementary strand 220 that is complementary to the second and third regions 222, 223 of the reporter oligonucleotide 214. The complementary strand 220 also includes a toehold sequence 221' and the fluorophore molecule 28.

The authentication assay 200 further includes a sample 224 of the authenticity label 236 collected from the product 38. The sample 224 includes at least one DNA taggant 226, as illustrated in FIG. 8B. The DNA taggant 226 is a single strand of DNA having a sequence 221 that is complementary to the toehold sequence 221' of the complementary strand, and a same sequence as the first and second regions 222, 223 of the reporter oligonucleotide 214. The sample 224, including the DNA taggant 226, may be added to a liquid reagent and incubated with the substrate 10.

During incubation, the DNA taggant 226 binds to the toehold sequence 221' of the complementary strand 220 via the sequence 221 that is complementary to the toehold sequence 221', as illustrated in FIG. 8B. This binding initiates a toehold-mediated DNA strand displacement reaction, in which displaces the complementary strand 220 from the reporter oligonucleotide 214. The complementary strand 220, including the fluorophore molecule 28, binds to the DNA taggant 226 and is removed from the assay when the sample 224 is washed from the substrate 10 with a neutral buffer. As such, as shown in FIG. 8C, no fluorescence is detected when the substrate 10 is exposed to light 30.

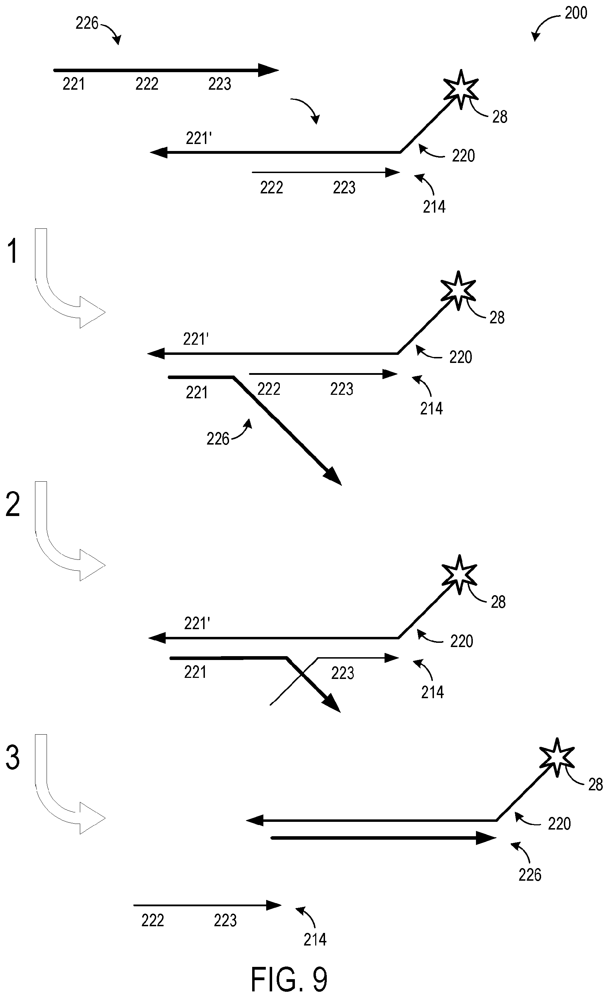

FIG. 9 illustrates a diagram of the displacement of the fluorophore-labeled complementary strand 220 from the reporter oligonucleotide 214. It will be appreciated that the DNA strands are shown to have directionality from the 5' to the 3' end, as is conventional. As described above and shown in FIG. 9, the 5' toehold sequence 221 of the DNA taggant 226 binds to the 3' toehold sequence 221' of the complementary strand 220 (indicated by arrow 1). This binding initiates a toehold-mediated DNA strand displacement reaction, in which the complementary strand 220 is displaced from the reporter oligonucleotide 214 (indicated by arrow 2). The complementary strand 220, including the fluorophore molecule 28, binds to the DNA taggant 226 and is removed from the authentication assay 200 (indicated by arrow 3).

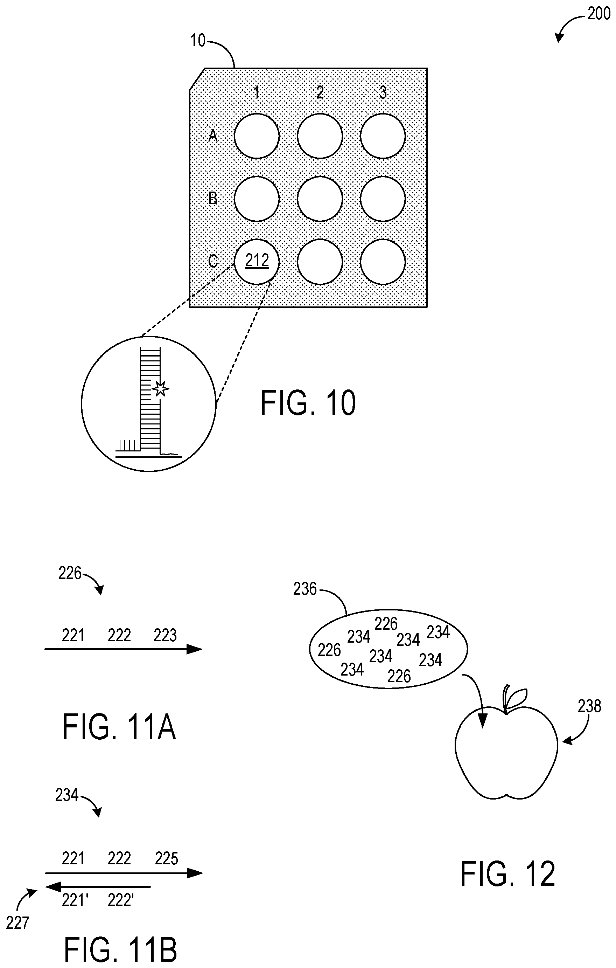

FIG. 10 shows an example of the reporter oligonucleotide 214 bound to the substrate 10 at the assay location 212 of the authentication assay 200. As with the authentication assay 100, the authentication assay 200 is configured as a spot assay, and a notch or the like may be formed in a corner of the substrate 10 for the purpose of orientation. An example reporter oligonucleotide 214 is depicted in the enlarged illustration of the assay location 12 in FIG. 10. As described above, the reporter oligonucleotides 214 may be deposited, i.e., printed, on the substrate 10 using an inkjet printer or the like, or they may be deposited using a micropipette or another suitable instrument capable of accurately transferring precise quantities of the components.

FIGS. 11A and 11B are diagrams of the DNA taggant 226 and a decoy DNA taggant 234, respectively, of the authentication assay 200. As described above, the DNA taggant 226 is a single strand of DNA that binds to the toehold sequence 221' of the complementary strand 220 during toehold-mediated DNA strand displacement reaction and has a same sequence as the first and second regions 222, 223 of the reporter oligonucleotide 214. The decoy DNA taggant 234 has a toehold binding sequence 221 and a same sequence as the universal first region 222 of the reporter oligonucleotide 214. However, the decoy DNA taggant 234 has a different sequence 225 from that of the unique sequence in the second region 223 of the reporter oligonucleotide 214. Additionally, the decoy DNA taggant 234 is prehybridized with a blocking strand 227 having a same sequence as the toehold sequence 221' and first region of the complementary strand. As such, the first region 221 of the decoy DNA taggant 234 does not bind the toehold region 221' of the reporter oligonucleotide 214, nor is it complementary to the second region of the complementary strand 220, thereby preventing the toehold-mediated DNA strand displacement reaction.

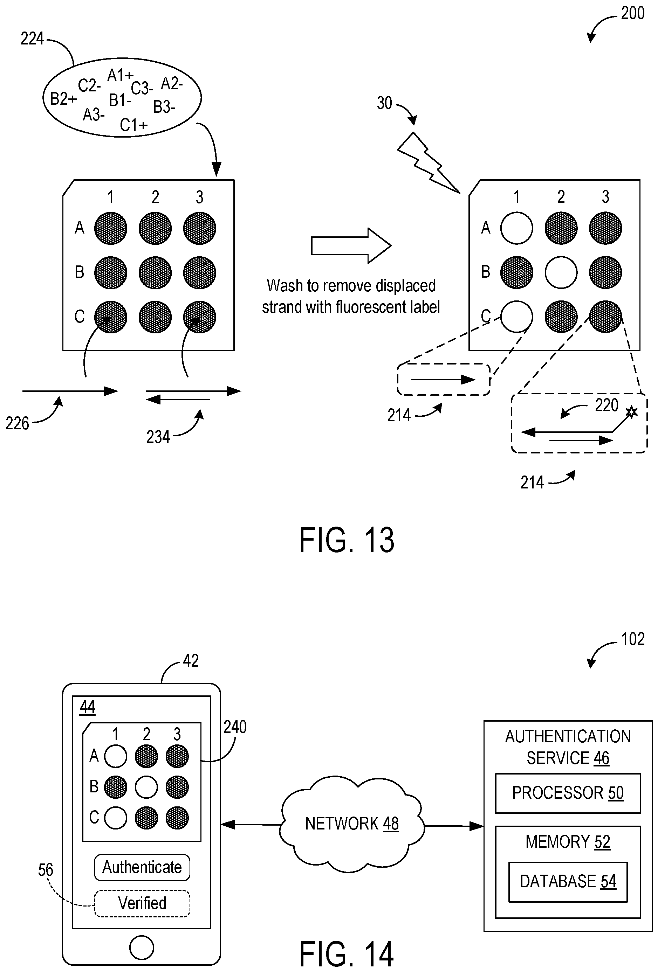

As with the authentication assay 100, the addition of the DNA taggants 226 and decoy DNA taggants 234 to the reporter oligonucleotides 214 at each assay location 212 create a fluorescent authenticity pattern on the substrate 10 upon excitation of the fluorophore molecules 28. However, as described below with reference to FIG. 13, since the fluorophore-labeled reporter oligonucleotides 214 are configured to fluoresce upon excitation prior to addition of the sample 224 and the DNA taggants 226, the lack of fluorescence at an assay location 212 indicates a positive result where a toehold-mediated DNA strand displacement reaction has occurred.

The addition of the authenticity label 236 to a product 238 is illustrated in FIG. 12. As discussed above, the product may be a commercial product, such as a high-value item, currency, food or agricultural products, or the like. As discussed above, binary data may be encoded in the DNA taggants, which enables the authenticity labels to be used for tracking and accountability. This implementation is particularly useful with food and agricultural products, as the DNA taggants may be designed to indicate a source of the product, as well as any treatments or special processes the product has undergone along the supply chain, such as insecticide treatment or lack thereof, for example. Additionally, labeling agricultural products enables the products to be tracked and identified in case of a recall due to contamination or the like, thereby providing information that can be utilized in regulatory and/or quality control aspects.

In the example shown in FIG. 12, the authenticity label 236 is used to label an agricultural product, specifically an apple 238. In this implementation, the authenticity label 236 may be included in a coating that is applied to a surface of the product. For example, when labeling an apple, the authenticity label may be embedded in a wax coating that is applied to (e.g., sprayed on) several apples at once.

FIG. 13 illustrates analyzing the sample 224 with the authentication assay 200, and the resulting pattern of the authentication assay 200 upon excitation of the fluorophore molecules 28. As described above, the sample 224 of the authenticity label 236 may be swabbed from the product 238 and placed in an amount of liquid reagent to distribute the DNA taggants 226 throughout the sample 224. As described above with reference to FIG. 6 and the authentication assay 100, the DNA taggants contained in the sample 224 of the authenticity label 236 are indicated by "A1+," "B2+," and "C1+," and the decoy DNA taggants are indicated by "A2-," "A3-," "B1-," "B3-," "C2-," and "C3-," corresponding to the indicated assay locations 12. As an example, when the DNA taggant 226 binds to the complementary strand 220 in assay location C1, the complementary strand 220, including the fluorophore molecule 28, is displaced from the reporter oligonucleotide 214 and washed away. As such, that assay location does not fluoresce upon excitation with a light source. In contrast, the decoy DNA taggant 234 does not bind to the complementary strand 220 in assay location C3, so the complementary strand 220, including the fluorophore molecule 28, remains attached to the reporter oligonucleotide 214 and fluoresces upon excitation, as indicated by the shading in assay location C3. Thus, the substrate 10 displays a fluorescent authenticity pattern 240 after incubation of the substrate 10 with the sample 224.

As indicated above, the authenticity label 236 may be configured to encode binary data. For example, the DNA strands of the taggants that include the toehold-binding, first, and second regions 221, 222, 223 may be defined as DNAbits. In the assay 200 described herein, the DNA taggants 226 may encode 1s of DNAbits, and the decoy DNA taggants 234, which prevent toehold-mediated strand displacement and removal of the fluorophore molecule 28 from the reporter oligonucleotide 214, may encode 0s of DNAbits. The binding of the DNA taggants 226 and lack of binding of the decoy DNA taggants 234 to reporter oligonucleotides 214 bound to different assay locations 212 result conversion of the 1s and 0s encoded in the DNAbits to a pattern of fluorescence, thereby enabling the authenticity of a product, as well as additional data, to be determined in a single, multiplex assay that simplifies the reading process.

As described above with reference to FIG. 7 and shown in FIG. 13, the system 102 for analyzing the authentication assay may be used to verify the authenticity label 236. The user may capture an image of the fluorescent authenticity pattern 240 with a mobile computing device 42 and transmit the fluorescent authenticity pattern 240 to an authentication server 46 to verify the authenticity label 236 based on the fluorescent authenticity pattern 240. The user may additionally or alternatively use a pattern key to validate the fluorescent authenticity pattern 240 and authenticate the product 238.

A potential downfall of using DNA taggants to authenticate products is that DNA can be copied via PCR and/or sequenced by anyone with access to suitable equipment and reagents. This may lead to forgery of the labels and application of forged labels to counterfeit products. However, as discussed above, the combination of DNA taggants and decoy DNA taggants in the authenticity labels 36, 236 described herein make it difficult to mimic the DNA taggants. As such, attempts to forge the DNA taggants would result in the inability reproduce the fluorescent authenticity pattern on the substrate 10, thereby protecting the authenticity labels 36, 236 against counterfeiting and forgery. Additionally, each label may contain millions of copies of DNA taggants and decoy DNA taggants, thereby providing a high level of redundancy.

As the information encoded in the DNA taggants is encoded by both the sequences and the hybridization state, it is not possible to determine the information, and thus the fluorescent authenticity pattern, by sequencing alone. Additionally, the DNA taggants are designed to be difficult to sequence. FIGS. 15A and 15B illustrate how the DNA taggants 26 and 226 are unable to be mimicked via PCR. In FIG. 15A, the authenticity label 36 of the authentication assay 100 contains a combination of DNA taggants 26A, 26B, 26C and decoy DNA taggants 34A, 34B. When the authenticity label 36 is subjected to a heating cycle of PCR, the DNA strands of the DNA taggants 26 and decoy DNA taggants 34 separate such that there is a mixture of short strands labeled with the fluorophore molecule 28 from the DNA taggants 26 and unlabeled short strands from the decoy DNA taggants 34. During the subsequent cooling cycle of PCR, the short strands anneal to longer strands, thereby yielding authenticity label 36' with a mixture of fluorophore-labeled DNA taggants, unlabeled (i.e., incorrect) DNA taggants, unlabeled decoy DNA taggants, and fluorophore-labeled (i.e., incorrect) decoy DNA taggants. As the DNA taggants 26A, 26B, 26C and decoy DNA taggants 34A, 34B are configured to bind to unique reporter oligonucleotides 14 bound to each assay location 12, incubation of the mixed sample 36' results in fluorescence at each assay location 12, thereby failing to produce the fluorescent authenticity pattern 40. For example, the decoy DNA taggant 34A, which hybridized with a short strand labeled with the fluorophore molecule 28 during the cooling cycle, may bind to the correct assay location 12, but it would incorrectly emit light upon excitation.

In FIG. 15B the authenticity label 236 of the authentication assay 200 contains a combination of DNA taggants 226A, 226B, 226C and decoy DNA taggants 234A, 234B. As described with reference to FIG. 15A, the heating and cooling cycles of PCR result in an authenticity label 236' that is a mixture of correct and incorrect DNA taggants and decoy DNA taggants. When the substrate 10 is incubated with the mixed sample 236', each assay location 12 emits at least some level of fluorescence due to the displacement and lack of displacement of the fluorophore-labeled complementary strand 220, rather than the fluorescent authenticity pattern 240.

To further prevent fraudulent sequencing of the DNA taggants and reporter oligonucleotides described herein, the DNA strands may be chemically modified. For example, a phosphoramidite C3 spacer can be incorporated into the sequence of the DNA taggant and/or the reporter oligonucleotide, which introduces a long hydrophilic spacer arm to which fluorophores or other molecules may be attached. A C3 spacer can be added to one or both ends of the DNA taggant and/or the reporter oligonucleotide to modify the 5' phosphate group and/or the 3' hydroxyl group. Such modification blocks the ligation of DNA sequencing adapters to the DNA taggant and/or the reporter oligonucleotide, thereby preventing sequencing of the DNA taggant and/or the reporter oligonucleotide. Additional chemical modifications that alter the 3' and/or 5' ends of the DNA taggants and/or reporter oligonucleotides to prevent sequencing may include adding phosphorothioate (PS) bonds to the 5' and/or 3' ends, phosphorylating the 3' ends, and/or linking an inverted dideoxythymidine (dT) to the 3' and/or 5' ends. Additionally, such chemical modifications may also be implemented to reduce degradation of the DNA taggants and the reporter oligonucleotides by exonucleases and other enzymes. It will be appreciated that the chemical modifications described herein are not intended to be limiting, and that other suitable chemical modifications may be employed to prevent sequencing and reproduction of the DNA taggants and/or reporter oligonucleotides to create fraudulent authenticity labels, as well as to prevent degradation of the DNA taggants and/or reporter oligonucleotides.

As an additional security measure for ensuring the fluorescent authenticity patterns described herein remain highly challenging to replicate, the sequences and patterns may be periodically changed. For example, the assay locations containing reporter oligonucleotides configured to undergo toehold-mediated DNA strand displacement upon incubation with a sample may be moved or rotated. Additionally or alternatively, the sequences of homology may be changed. In some implementations, the authenticity label may contain a plurality of DNA taggants, but the substrate is configured to detect only a subset of the DNA taggants.

Mimicking the fluorescent authenticity pattern via brute force is challenging, as the number of possible combinations of fluorescence is 2^{number of sequences}. Additionally, at the server level, the processor of the authentication server may be configured to recognize multiple attempts using different fluorescent authenticity patterns to verify a product from a same user and implement a computational warning after a threshold number of attempts to authenticate a product using different fluorescent authentication patterns.

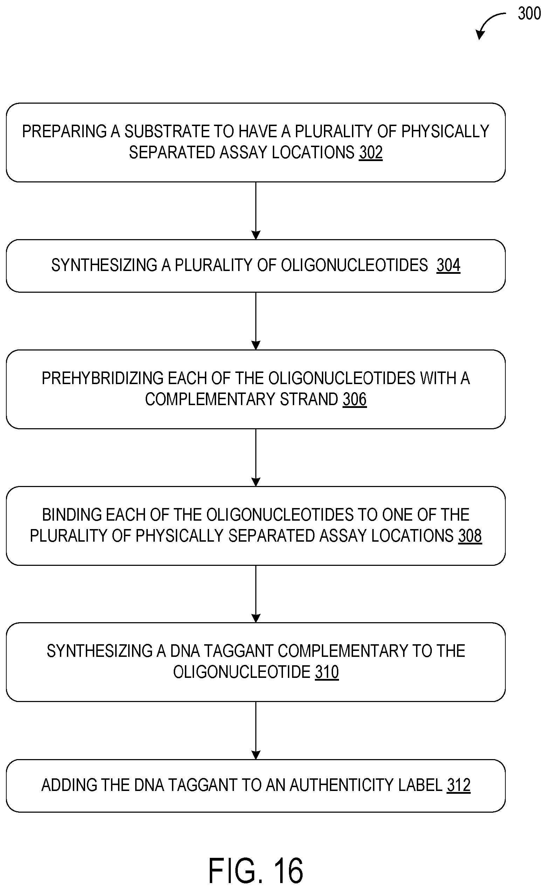

FIG. 16 is a flowchart of a method 300 for manufacturing an authentication assay using embedded deoxyribonucleic acid (DNA) taggants according to an example configuration of the present disclosure. At step 302, the method 300 may include preparing a substrate to have a plurality of physically separated assay locations. As described above, when the substrate is a nitrocellulose membrane, the physically separated assay locations may be formed by depositing a pattern of hydrophobic barriers on the nitrocellulose membrane with a wax-based printer.

Continuing from step 302 to step 304, the method 300 may include synthesizing a plurality of reporter oligonucleotides. Each of the reporter oligonucleotides may have a first region including a single-stranded toehold region, a second region including a universal sequence that is common among each of the reporter oligonucleotides, and a third region including a unique sequence that is different for each of the reporter oligonucleotides.

Proceeding from step 304 to step 306, the method 300 may include prehybridizing the second and third regions of each reporter oligonucleotide of the plurality of reporter oligonucleotides with a complementary strand. The complementary strand may have a sequence that is complementary to the second and third regions of each reporter oligonucleotide.

Advancing from step 306 to step 308, the method 300 may include binding each reporter oligonucleotide to one of the plurality of physically separated assay locations. The binding may be facilitated by an anchor region included in each reporter oligonucleotide. As such, each assay location may include a reporter oligonucleotide with a unique sequence in the third region Continuing from step 308 to step 310, the method 300 may include synthesizing at least one DNA taggant. The DNA taggant may have a sequence complementary to the first and second regions of each of the reporter oligonucleotides, and complementary to the third region of at least one reporter oligonucleotide bound to an assay location of the plurality of assay locations. The DNA taggant may further include a fluorophore molecule that is configured to emit light upon excitation.

Proceeding from step 310 to step 312, the method 300 may include adding the DNA taggant to an authenticity label that is configured to verify an authenticity of a product. As described above, when the substrate is incubated with a sample of the authenticity label, the DNA taggant binds to the toehold region of the reporter oligonucleotide. This binding initiates a toehold-mediated DNA strand displacement reaction thereby exchanging the complementary strand of the at least one reporter oligonucleotide with the at least one DNA taggant. As the DNA taggant includes the fluorophore molecule, a user can verify an authenticity of the product via a pattern of emitted light at one or more assay locations of the plurality of assay locations upon excitation of the fluorophore molecule attached to the DNA taggant.

The embodiments described above may be used to verify the authenticity of a product quickly and accurately. The authentication assays are configured to display patterns of fluorescence specific to respective products without the need for a quenching molecule, thereby providing a cost-effective mechanism for using fluorescence technology to label products. The cost of manufacturing the authenticity assay is further reduced in embodiments in which the fluorophore molecule is configured to label the reporter oligonucleotide anchored to the substrate, as fewer fluorophore molecules are needed compared to embodiments in which the DNA taggants are fluorescently labeled. Additionally, the authenticity label may contain a combination of DNA taggants and decoy DNA taggants, making it difficult to mimic the DNA taggants and reproduce the fluorescent authenticity pattern on the substrate, and thereby protecting the authenticity labels against counterfeiting and forgery. Furthermore, the described authenticity labels can be embedded in a variety of solid, powdered, and liquid materials, and each label includes millions of copies of DNA taggants, thereby providing a high level of redundancy. In addition to verifying the authenticity of products, binary data may be encoded in the DNA taggants, which enables the authenticity labels to be used for tracking and accountability in supply chains. The DNA taggants may be designed to indicate a source of the product, materials used in the manufacture of the product, and any treatments or special processes a product or commodity has undergone. With these technical aspects, the authentication assay embodiments described herein provide numerous advantages over existing authenticity label technology.

The following paragraphs provide additional description of aspects of the present disclosure. According to one aspect an authentication assay using embedded deoxyribonucleic acid (DNA) taggants is provided, comprising a substrate having a plurality of physically separated assay locations, each assay location of the plurality of assay locations including a reporter oligonucleotide bound to the substrate via an anchor region, and a sample of an authenticity label collected from a product. In this aspect, each reporter oligonucleotide bound to each assay location of the plurality of assay locations includes a first region, a second region, and a third region, the first region of each reporter oligonucleotide includes a single-stranded toehold sequence that is common among each of the reporter oligonucleotides bound to each assay location of the plurality of assay locations, the second region of each reporter oligonucleotide includes a universal sequence that is common among each of the reporter oligonucleotides bound to each assay location of the plurality of assay locations, the third region of each reporter oligonucleotide includes a unique sequence that is different for each of the reporter oligonucleotides bound to each assay location of the plurality of assay locations, the second and third regions of each reporter oligonucleotide are prehybridized with a complementary strand having a sequence that is complementary to the second and third regions of each reporter oligonucleotide, the sample includes at least one DNA taggant having a sequence complementary to the first and second regions of each of the reporter oligonucleotides, and complementary to the third region of at least one reporter oligonucleotide bound to an assay location of the plurality of assay locations, the at least one DNA taggant including a fluorophore molecule configured to emit light upon excitation, incubation of the substrate with the sample results in binding of the at least one DNA taggant to the at least one reporter oligonucleotide, thereby initiating a toehold-mediated DNA strand displacement reaction that exchanges the complementary strand of the at least one reporter oligonucleotide for the at least one DNA taggant including the fluorophore molecule, and excitation of the fluorophore molecule attached to the DNA taggant causes the fluorophore molecule to emit light, thereby resulting in a pattern of emitted light at one or more assay locations. In an example configuration of the authentication assay according to this aspect, the at least one DNA taggant is one of a plurality of DNA taggants included in the sample, and each DNA taggant of the plurality of DNA taggants included in the sample has a third region with a unique sequence complementary to a third region of a reporter oligonucleotide bound to one of the assay locations of the plurality of assay locations.

In an example configuration of the authentication assay according to this aspect, the sample additionally includes at least one decoy DNA taggant having a third region with a unique sequence complementary to a reporter oligonucleotide bound to one of the assay locations of the plurality of assay locations, the at least one decoy DNA taggant lacking a fluorophore molecule.

A system for analyzing the authentication assay of this aspect is also provided. In an example configuration, the system comprises a light emitting device configured to emit light at a wavelength that causes excitation of the fluorophore molecule attached to the DNA taggant; an optical reader configured to capture the pattern of emitted light; and a computing device configured to receive data encoding the pattern of emitted light from the optical reader, perform a verification action on the pattern of emitted light, and output a verification result.

In an example configuration of the authentication assay according to this aspect, the light emitting device is a handheld light, the optical reader is a camera-equipped mobile computing device, and the computing device is a server configured to receive the data encoding the pattern of emitted light over a computer network from the mobile computing device.

In an example configuration of the authentication assay according to this aspect, the authenticity label is included in a coating applied to a surface of the product.

In an example configuration of the authentication assay according to this aspect, when the product is a liquid product, the authenticity label is added directly to the product.

According to another aspect, a method for manufacturing an authentication assay using embedded deoxyribonucleic acid (DNA) taggants is provided, the method comprising: preparing a substrate to have a plurality of physically separated assay locations; synthesizing a plurality of reporter oligonucleotides, each of which has a first region including a single-stranded toehold sequence that is common among each of the reporter oligonucleotides, a second region including a universal sequence that is common among each of the reporter oligonucleotides, and a third region including a unique sequence that is different for each of the reporter oligonucleotides; prehybridizing the second and third regions of each reporter oligonucleotide of the plurality of reporter oligonucleotides with a complementary strand having a sequence that is complementary to the second and third regions of each reporter oligonucleotide; binding, via an anchor region, each reporter oligonucleotide of the plurality of reporter oligonucleotides to one of the plurality of physically separated assay locations such that each assay location includes a reporter oligonucleotide with a unique sequence in the third region; synthesizing at least one DNA taggant to have a sequence complementary to the first and second regions of each of the reporter oligonucleotides, and complementary to the third region of at least one reporter oligonucleotide bound to an assay location of the plurality of assay locations, the at least one DNA taggant including a fluorophore molecule configured to emit light upon excitation; and adding the at least one DNA taggant to an authenticity label configured to verify an authenticity of a product, wherein incubation of the substrate with a sample of the authenticity label results in binding of the at least one DNA taggant to the at least one reporter oligonucleotide, thereby initiating a toehold-mediated DNA strand displacement reaction that exchanges the complementary strand of the at least one reporter oligonucleotide for the at least one DNA taggant including the fluorophore molecule, and excitation of the fluorophore molecule attached to the DNA taggant causes the fluorophore molecule to emit light, thereby resulting in a pattern of emitted light at one or more assay locations. In an example configuration of the method according to this aspect, the at least one DNA taggant is one of a plurality of DNA taggants included in the sample, and the method further comprises synthesizing each DNA taggant of the plurality of DNA taggants included in the sample to have a third region with a unique sequence complementary to a reporter oligonucleotide bound to one of the assay locations of the plurality of assay locations.

In an example configuration of the method according to this aspect, the method further comprises synthesizing at least one decoy DNA taggant having a third region with a unique sequence complementary to a reporter oligonucleotide bound to one of the assay locations of the plurality of assay locations, the at least one decoy DNA taggant lacking a fluorophore molecule; and including the at least one decoy DNA taggant in the authenticity label.

In an example configuration of the method according to this aspect, the method further comprises exciting the fluorophore molecule attached to the DNA taggant with a light emitting device, and capturing the pattern of emitted light with an optical reader.

In an example configuration of the method according to this aspect, the method further comprises receiving an authentication result output from a computing device configured to receive data encoding the pattern of emitted light from the optical reader and perform an authentication action on the pattern of emitted light.

In an example configuration of the method according to this aspect, the method further comprises including the authenticity label in a coating applied to a surface of the product.

In an example configuration of the method according to this aspect, the method further comprises, when the product is a liquid product, adding the authenticity label to the product.

According to another aspect, an authentication assay using embedded deoxyribonucleic acid (DNA) taggants is provided, the assay comprising: a substrate having a plurality of physically separated assay locations, each assay location of the plurality of assay locations including a reporter oligonucleotide bound to the substrate via an anchor region; and a sample of an authenticity label collected from a product, wherein each reporter oligonucleotide bound to each assay location of the plurality of assay locations includes a first region and a second region, the first region of each reporter oligonucleotide includes a universal sequence that is common among each of the reporter oligonucleotides bound to each assay location of the plurality of assay locations, the second region of each reporter oligonucleotide includes a unique sequence that is different for each of the reporter oligonucleotides bound to each assay location of the plurality of assay locations, the first and second regions of each reporter oligonucleotide are prehybridized with a complementary strand having a sequence that is complementary to the first and second regions of each reporter oligonucleotide, the complementary strand including a toehold sequence and a fluorophore molecule, the sample includes at least one DNA taggant having a same sequence as the first and second regions of at least one reporter oligonucleotide bound to an assay location of the plurality of assay locations, and a toehold sequence complementary to the toehold sequence of the complementary strand, incubation of the substrate with the sample results in binding of the at least one DNA taggant to the complementary strand, thereby initiating a toehold-mediated DNA strand displacement reaction that displaces the complementary strand, including the fluorophore molecule, from the at least one reporter oligonucleotide, and exposure of the substrate to light configured to excite fluorophore molecules produces a pattern of emitted light at one or more assay locations, the pattern including an absence of emitted light at one or more assay locations of the plurality of assay locations due to release of the complementary strand, including the fluorophore molecule.

In an example configuration of the authentication assay according to this aspect, the at least one DNA taggant is one of a plurality of DNA taggants included in the sample, and each DNA taggant of the plurality of DNA taggants included in the sample has a second region with a same unique sequence as a second region of a reporter oligonucleotide bound to one of the assay locations of the plurality of assay locations.

In an example configuration of the authentication assay according to this aspect, the sample additionally includes at least one decoy DNA taggant having a same universal sequence as the first region of at least one reporter oligonucleotide, a different sequence than a unique sequence of a second region of the at least one reporter oligonucleotide, and a toehold sequence complementary to the toehold sequence of the complementary strand, and the DNA taggant is prehybridized with a blocking strand having a same sequence as the toehold sequence and first region of the complementary strand, thereby blocking the toehold-mediated DNA strand displacement reaction that displaces the complementary strand, including the fluorophore molecule, from the at least one reporter oligonucleotide.

A system is provided for analyzing the authentication assay of this aspect. In one example configuration, the system comprises a light emitting device configured to emit light at a wavelength that causes excitation of a fluorophore molecule attached to the complementary strand; an optical reader configured to capture the pattern of emitted light, including the absence of emitted light at one or more assay locations; and a computing device configured to receive data encoding the pattern of emitted light from the optical reader, perform a verification action on the pattern of emitted light, and output a verification result, wherein the light emitting device is a handheld light, the optical reader is a camera-equipped mobile computing device, and the computing device is a server configured to receive the data encoding the pattern of emitted light over a computer network from the mobile computing device.

In an example configuration of the authentication assay according to this aspect, the authenticity label is included in a coating applied to a surface of the product.

In an example configuration of the authentication assay according to this aspect, when the product is a liquid product, the authenticity label is added directly to the product.

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

## Claims

1. An authentication assay (100) using embedded deoxyribonucleic acid, DNA, taggants, the assay comprising:
a substrate (10) having a plurality of physically separated assay locations (12), each assay location (12) of the plurality of assay locations (12) including a reporter oligonucleotide (14) bound to the substrate (10) via an anchor region (18); and
a sample of an authenticity label collected from a product, wherein
each reporter oligonucleotide (14) bound to each assay location (12) of the plurality of assay locations (12) includes a first region (21), a second region (22), and a third region (23),
the first region (21) of each reporter oligonucleotide (14) includes a single-stranded (20) toehold sequence that is common among each of the reporter oligonucleotides (14) bound to each assay location (12) of the plurality of assay locations (12),
the second region (22) of each reporter oligonucleotide (14) includes a universal sequence that is common among each of the reporter oligonucleotides (14) bound to each assay location (12) of the plurality of assay locations (12),
the third region (23) of each reporter oligonucleotide (14) includes a unique sequence that is different for each of the reporter oligonucleotides (14) bound to each assay location (12) of the plurality of assay locations (12),
the second and third regions (22, 23) of each reporter oligonucleotide (14) are prehybridized with a complementary strand (20) having a sequence that is complementary to the second and third regions (22, 23) of each reporter oligonucleotide (14),
the sample includes at least one DNA taggant (26) having a sequence complementary to the first and second regions (21, 22) of each of the reporter oligonucleotides (14), and complementary to the third region (23) of at least one reporter oligonucleotide (14) bound to an assay location (12) of the plurality of assay locations (12), the at least one DNA taggant (26) including a fluorophore molecule configured to emit light upon excitation,
incubation of the substrate (10) with the sample results in binding of the at least one DNA taggant (26) to the at least one reporter oligonucleotide (14), thereby initiating a toehold-mediated DNA strand (20) displacement reaction that exchanges the complementary strand (20) of the at least one reporter oligonucleotide (14) for the at least one DNA taggant (26) including the fluorophore molecule, and
excitation of the fluorophore molecule attached to the DNA taggant (26) causes the fluorophore molecule to emit light, thereby resulting in a pattern of emitted light at one or more assay locations (12).

2. The authentication assay (100) of claim 1, wherein
the at least one DNA taggant (26) is one of a plurality of DNA taggants (26) included in the sample, and
each DNA taggant (26) of the plurality of DNA taggants (26) included in the sample has a third region (23) with a unique sequence complementary to a third region (23) of a reporter oligonucleotide (14) bound to one of the assay locations (12) of the plurality of assay locations (12).

3. The authentication assay (100) of claim 1, wherein
the sample additionally includes at least one decoy DNA taggant (26) having a third region (23) with a unique sequence complementary to a reporter oligonucleotide (14) bound to one of the assay locations (12) of the plurality of assay locations (12), the at least one decoy DNA taggant (26) lacking a fluorophore molecule.

4. A system (102) configured for analyzing the authentication assay (100) of claim 1, comprising:
a light emitting device configured to emit light at a wavelength that causes excitation of the fluorophore molecule attached to the DNA taggant (26);
an optical reader (42) configured to capture the pattern of emitted light; and
a computing device configured to receive data encoding the pattern of emitted light from the optical reader (42), perform a verification action on the pattern of emitted light, and output a verification result.

5. The system (102) of claim 4, wherein
the light emitting device is a handheld light,
the optical reader (42) is a camera-equipped mobile computing device, and
the computing device is a server configured to receive the data encoding the pattern of emitted light over a computer network from the mobile computing device.

6. A method for manufacturing an authentication assay (100) using embedded deoxyribonucleic acid, DNA, taggants, the method comprising:
Preparing (302) a substrate (10) to have a plurality of physically separated assay locations (12);
synthesizing (304) a plurality of reporter oligonucleotides (14), each of which has a first region (21) including a single-stranded (20) toehold sequence that is common among each of the reporter oligonucleotides (14), a second region (22) including a universal sequence that is common among each of the reporter oligonucleotides (14), and a third region (23) including a unique sequence that is different for each of the reporter oligonucleotides (14);
prehybridizing (306) the second and third regions (22, 23) of each reporter oligonucleotide (14) of the plurality of reporter oligonucleotides (14) with a complementary strand (20) having a sequence that is complementary to the second and third regions (22, 23) of each reporter oligonucleotide (14);
binding (308), via an anchor region (18), each reporter oligonucleotide (14) of the plurality of reporter oligonucleotides (14) to one of the plurality of physically separated assay locations (12) such that each assay location (12)includes a reporter oligonucleotide (14) with a unique sequence in the third region (23);
synthesizing (310) at least one DNA taggant (26) to have a sequence complementary to the first and second regions (21, 22) of each of the reporter oligonucleotides (14), and complementary to the third region (23) of at least one reporter oligonucleotide (14) bound to an assay location (12) of the plurality of assay locations (12), the at least one DNA taggant (26) including a fluorophore molecule configured to emit light upon excitation; and
adding (312) the at least one DNA taggant (26) to an authenticity label configured to verify an authenticity of a product, wherein
incubation of the substrate (10) with a sample of the authenticity label results in binding of the at least one DNA taggant (26) to the at least one reporter oligonucleotide (14), thereby initiating a toehold-mediated DNA strand (20) displacement reaction that exchanges the complementary strand (20) of the at least one reporter oligonucleotide (14) for the at least one DNA taggant (26) including the fluorophore molecule, and
excitation of the fluorophore molecule attached to the DNA taggant (26) causes the fluorophore molecule to emit light, thereby resulting in a pattern of emitted light at one or more assay locations (12).

7. The method of claim 6, wherein
the at least one DNA taggant (26) is one of a plurality of DNA taggants (26) included in the sample, and
the method further comprises synthesizing each DNA taggant (26) of the plurality of DNA taggants (26) included in the sample to have a third region (23) with a unique sequence complementary to a reporter oligonucleotide (14) bound to one of the assay locations (12) of the plurality of assay locations (12).

8. The method of claim 6, the method further comprising:
synthesizing at least one decoy DNA taggant (26) having a third region (23) with a unique sequence complementary to a reporter oligonucleotide (14) bound to one of the assay locations (12) of the plurality of assay locations (12), the at least one decoy DNA taggant (26) lacking a fluorophore molecule; and
including the at least one decoy DNA taggant (26) in the authenticity label.

9. The method of claim 6, the method further comprising:
exciting the fluorophore molecule attached to the DNA taggant (26) with a light emitting device; and
capturing the pattern of emitted light with an optical reader (42).

10. The method of claim 9, the method further comprising:
receiving an authentication result output from a computing device configured to receive data encoding the pattern of emitted light from the optical reader (42) and perform an authentication action on the pattern of emitted light.

11. An authentication assay (100) using embedded deoxyribonucleic acid, DNA, taggants, the assay comprising:
a substrate (10) having a plurality of physically separated assay locations (12), each assay location (12) of the plurality of assay locations (12) including a reporter oligonucleotide (14) bound to the substrate (10) via an anchor region (18); and
a sample of an authenticity label collected from a product, wherein
each reporter oligonucleotide (14) bound to each assay location (12) of the plurality of assay locations (12) includes a first region (21) and a second region (22),
the first region (21) of each reporter oligonucleotide (14) includes a universal sequence that is common among each of the reporter oligonucleotides (14) bound to each assay location (12) of the plurality of assay locations (12),
the second region (22) of each reporter oligonucleotide (14) includes a unique sequence that is different for each of the reporter oligonucleotides (14) bound to each assay location (12) of the plurality of assay locations (12),
the first and second regions (21, 22) of each reporter oligonucleotide (14) are prehybridized with a complementary strand (20) having a sequence that is complementary to the first and second regions (21, 22) of each reporter oligonucleotide (14), the complementary strand (20) including a toehold sequence and a fluorophore molecule,
the sample includes at least one DNA taggant (26) having a same sequence as the first and second regions (21, 22) of at least one reporter oligonucleotide (14) bound to an assay location (12) of the plurality of assay locations (12), and a toehold sequence complementary to the toehold sequence of the complementary strand (20),
incubation of the substrate (10) with the sample results in binding of the at least one DNA taggant (26) to the complementary strand (20), thereby initiating a toehold-mediated DNA strand (20) displacement reaction that displaces the complementary strand (20), including the fluorophore molecule, from the at least one reporter oligonucleotide (14), and
exposure of the substrate (10) to light configured to excite fluorophore molecules produces a pattern of emitted light at one or more assay locations (12), the pattern including an absence of emitted light at one or more assay locations (12) of the plurality of assay locations (12) due to release of the complementary strand (20), including the fluorophore molecule.

12. The authentication assay (100) of claim 11, wherein
the at least one DNA taggant (26) is one of a plurality of DNA taggants (26) included in the sample, and
each DNA taggant (26) of the plurality of DNA taggants (26) included in the sample has a second region (22) with a same unique sequence as a second region (22) of a reporter oligonucleotide (14) bound to one of the assay locations (12) of the plurality of assay locations (12).

13. The authentication assay (100) of claim 11, wherein
the sample additionally includes at least one decoy DNA taggant (26) having a same universal sequence as the first region (21) of at least one reporter oligonucleotide (14), a different sequence than a unique sequence of a second region (22) of the at least one reporter oligonucleotide (14), and a toehold sequence complementary to the toehold sequence of the complementary strand (20), and
the DNA taggant (26) is prehybridized with a blocking strand (20) having a same sequence as the toehold sequence and first region (21) of the complementary strand (20), thereby blocking the toehold-mediated DNA strand (20) displacement reaction that displaces the complementary strand (20), including the fluorophore molecule, from the at least one reporter oligonucleotide (14).

14. A system (102) configured for analyzing the authentication assay (100) of claim 11, comprising:
a light emitting device configured to emit light at a wavelength that causes excitation of a fluorophore molecule attached to the complementary strand (20);
an optical reader (42) configured to capture the pattern of emitted light, including the absence of emitted light at one or more assay locations (12); and
a computing device configured to receive data encoding the pattern of emitted light from the optical reader (42), perform a verification action on the pattern of emitted light, and output a verification result, wherein
the light emitting device is a handheld light,
the optical reader (42) is a camera-equipped mobile computing device, and
the computing device is a server configured to receive the data encoding the pattern of emitted light over a computer network from the mobile computing device.

15. The authentication assay (100) of claim 11, wherein
the authenticity label is included in a coating applied to a surface of the product.

## Patentansprüche

1. Authentifizierungsassay (100), welches eingebettete Desoxyribonukleinsäure-, DNA-, Marker verwendet, wobei das Assay Folgendes umfasst:
ein Substrat (10) mit einer Vielzahl von physikalisch getrennten Assaystellen (12), wobei jede Assaystelle (12) der Vielzahl von Assaystellen (12) ein Reporter-Oligonukleotid (14) beinhaltet, das über eine Ankerregion (18) an das Substrat (10) gebunden ist; und
eine Probe einer Authentizitätsmarkierung, die von einem Produkt gesammelt wurde, wobei
jedes Reporter-Oligonukleotid (14), das an jede Assaystelle (12) der Vielzahl von Assaystellen (12) gebunden ist, eine erste Region (21), eine zweite Region (22) und eine dritte Region (23) aufweist,
die erste Region (21) jedes Reporter-Oligonukleotids (14) eine einzelsträngige (20) Toehold-Sequenz aufweist, die unter jedem der Reporter-Oligonukleotide (14), die an jede Assaystelle (12) der Vielzahl von Assaystellen (12) gebunden sind, gemeinsam ist,
die zweite Region (22) jedes Reporter-Oligonukleotids (14) eine universelle Sequenz aufweist, die unter jedem der Reporter-Oligonukleotide (14), die an jede Assaystelle (12) der Vielzahl von Assaystellen (12) gebunden sind, gemeinsam ist,
die dritte Region (23) jedes Reporter-Oligonukleotids (14) eine eindeutige Sequenz aufweist, die für jedes der Reporter-Oligonukleotide (14), die an jede Assaystelle (12) der Vielzahl von Assaystellen (12) gebunden sind, unterschiedlich ist,
die zweite und dritte Region (22, 23) jedes Reporter-Oligonukleotids (14) mit einem komplementären Strang (20) vorhybridisiert sind, der eine Sequenz aufweist, die zu der zweiten und dritten Region (22, 23) jedes Reporter-Oligonukleotids (14) komplementär ist,
die Probe mindestens einen DNA-Marker (26) aufweist, der eine Sequenz aufweist, die zu der ersten und zweiten Region (21, 22) jedes der Reporter-Oligonukleotide (14) komplementär ist und zu der dritten Region (23) mindestens eines Reporter-Oligonukleotids (14), das an eine Assaystelle (12) der Vielzahl von Assaystellen (12) gebunden ist, komplementär ist, wobei der mindestens eine DNA-Marker (26) ein Fluorophormolekül aufweist, das dazu konfiguriert ist, bei Anregung Licht zu emittieren,
eine Inkubation des Substrats (10) mit der Probe zu einer Bindung des mindestens einen DNA-Markers (26) an das mindestens eine Reporter-Oligonukleotid (14) führt, wodurch eine Toehold-vermittelte DNA-Strang (20)-Verdrängungsreaktion initiiert wird, die den komplementären Strang (20) des mindestens einen Reporter-Oligonukleotids (14) gegen den mindestens einen DNA-Marker (26), der das Fluorophormolekül aufweist, austauscht, und
eine Anregung des Fluorophormoleküls, das an den DNA-Marker (26) gebunden ist, bewirkt, dass das Fluorophormolekül Licht emittiert, was zu einem Muster von emittiertem Licht an einer oder mehreren Assaystellen (12) führt.

2. Authentifizierungsassay (100) nach Anspruch 1, wobei
der mindestens eine DNA-Marker (26) einer einer Vielzahl von DNA-Markern (26) ist, die in der Probe enthalten sind, und
jeder DNA-Marker (26) der Vielzahl von DNA-Markern (26), die in der Probe enthalten sind, eine dritte Region (23) mit einer eindeutigen Sequenz aufweist, die zu einer dritten Region (23) eines Reporter-Oligonukleotids (14), das an eine der Assaystellen (12) der Vielzahl von Assaystellen (12) gebunden ist, komplementär ist.

3. Authentifizierungsassay (100) nach Anspruch 1, wobei
die Probe zusätzlich mindestens einen Decoy-DNA-Marker (26) aufweist, der eine dritte Region (23) mit einer eindeutigen Sequenz aufweist, die zu einem Reporter-Oligonukleotid (14), das an eine der Assaystellen (12) der Vielzahl von Assaystellen (12) gebunden ist, komplementär ist, wobei dem mindestens einen Decoy-DNA-Marker (26) ein Fluorophormolekül fehlt.

4. System (102), das zum Analysieren des Authentifizierungsassays (100) nach Anspruch 1 konfiguriert ist, umfassend:
eine lichtemittierende Vorrichtung, die dazu konfiguriert ist, Licht mit einer Wellenlänge zu emittieren, die eine Anregung des Fluorophormoleküls, das an den DNA-Marker (26) gebunden ist, bewirkt;
ein optisches Lesegerät (42), das dazu konfiguriert ist, das Muster von emittiertem Licht zu erfassen; und
eine Rechenvorrichtung, die dazu konfiguriert ist, Daten, die das Muster von emittiertem Licht codieren, von dem optischen Lesegerät (42) zu empfangen, eine Verifizierungsaktion an dem Muster von emittiertem Licht durchzuführen und ein Verifizierungsergebnis auszugeben.

5. System (102) nach Anspruch 4, wobei
die lichtemittierende Vorrichtung ein Handlicht ist,
das optische Lesegerät (42) eine mit einer Kamera ausgestattete mobile Rechenvorrichtung ist, und
die Rechenvorrichtung ein Server ist, der dazu konfiguriert ist, die Daten, die das Muster von emittiertem Licht codieren, über ein Computernetzwerk von der mobilen Rechenvorrichtung zu empfangen.

6. Verfahren zum Herstellen eines Authentifizierungsassays (100) unter Verwendung eingebetteter Desoxyribonukleinsäure-, DNA-, Marker, wobei das Verfahren Folgendes umfasst:
Vorbereiten (302) eines Substrats (10), so dass es eine Vielzahl von physikalisch getrennten Assaystellen (12) aufweist;
Synthetisieren (304) einer Vielzahl von Reporter-Oligonukleotiden (14), von denen jedes eine erste Region (21), die eine einzelsträngige (20) Toehold-Sequenz aufweist, die unter jedem der Reporter-Oligonukleotide (14) gemeinsam ist, eine zweite Region (22), die eine universelle Sequenz aufweist, die unter jedem der Reporter-Oligonukleotide (14) gemeinsam ist, und eine dritte Region (23), die eine eindeutige Sequenz aufweist, die für jedes der Reporter-Oligonukleotide (14) unterschiedlich ist, aufweist;
Vorhybridisieren (306) der zweiten und dritten Region (22, 23) jedes Reporter-Oligonukleotids (14) der Vielzahl von Reporter-Oligonukleotiden (14) mit einem komplementären Strang (20), der eine Sequenz aufweist, die zu der zweiten und dritten Region (22, 23) jedes Reporter-Oligonukleotids (14) komplementär ist;
Binden (308), über eine Ankerregion (18), jedes Reporter-Oligonukleotids (14) der Vielzahl von Reporter-Oligonukleotiden (14) an eine der Vielzahl von physikalisch getrennten Assaystellen (12), so dass jede Assaystelle (12) ein Reporter-Oligonukleotid (14) mit einer eindeutigen Sequenz in der dritten Region (23) aufweist;
Synthetisieren (310) mindestens eines DNA-Markers (26), so dass er eine Sequenz aufweist, die zu der ersten und zweiten Region (21, 22) jedes der Reporter-Oligonukleotide (14) komplementär ist und zu der dritten Region (23) mindestens eines Reporter-Oligonukleotids (14), das an eine Assaystelle (12) der Vielzahl von Assaystellen (12) gebunden ist, komplementär ist, wobei der mindestens eine DNA-Marker (26) ein Fluorophormolekül aufweist, das dazu konfiguriert ist, bei Anregung Licht zu emittieren; und
Hinzufügen (312) des mindestens einen DNA-Markers (26) zu einer Authentizitätsmarkierung, die dazu konfiguriert ist, eine Authentizität eines Produkts zu verifizieren, wobei
eine Inkubation des Substrats (10) mit einer Probe der Authentizitätsmarkierung zu einer Bindung des mindestens einen DNA-Markers (26) an das mindestens eine Reporter-Oligonukleotid (14) führt, wodurch eine Toehold-vermittelte DNA-Strang (20)-Verdrängungsreaktion initiiert wird, die den komplementären Strang (20) des mindestens einen Reporter-Oligonukleotids (14) gegen den mindestens einen DNA-Marker (26), der das Fluorophormolekül aufweist, austauscht, und
eine Anregung des Fluorophormoleküls, das an den DNA-Marker (26) gebunden ist, bewirkt, dass das Fluorophormolekül Licht emittiert, was zu einem Muster von emittiertem Licht an einer oder mehreren Assaystellen (12) führt.

7. Verfahren nach Anspruch 6, wobei
der mindestens eine DNA-Marker (26) einer einer Vielzahl von DNA-Markern (26) ist, die in der Probe enthalten sind, und
das Verfahren ferner das Synthetisieren jedes DNA-Markers (26) der Vielzahl von DNA-Markern (26), die in der Probe enthalten sind, umfasst, um eine dritte Region (23) mit einer eindeutigen Sequenz aufzuweisen, die zu einem Reporter-Oligonukleotid (14), das an eine der Assaystellen (12) der Vielzahl von Assaystellen (12) gebunden ist, komplementär ist.

8. Verfahren nach Anspruch 6, wobei das Verfahren ferner Folgendes umfasst:
Synthetisieren mindestens eines Decoy-DNA-Markers (26), der eine dritte Region (23) mit einer eindeutigen Sequenz aufweist, die zu einem Reporter-Oligonukleotid (14), das an eine der Assaystellen (12) der Vielzahl von Assaystellen (12) gebunden ist, komplementär ist, wobei dem mindestens einen Decoy-DNA-Marker (26) ein Fluorophormolekül fehlt; und
Einschließen des mindestens einen Decoy-DNA-Markers (26) in die Authentizitätsmarkierung.

9. Verfahren nach Anspruch 6, wobei das Verfahren ferner Folgendes umfasst:
Anregen des Fluorophormoleküls, das an den DNA-Marker (26) gebunden ist, mit einer lichtemittierenden Vorrichtung; und
Erfassen des Musters von emittiertem Licht mit einem optischen Lesegerät (42).

10. Verfahren nach Anspruch 9, wobei das Verfahren ferner Folgendes umfasst:
Empfangen eines Authentifizierungsergebnisses, das von einer Rechenvorrichtung ausgegeben wird, die dazu konfiguriert ist, Daten, die das Muster von emittiertem Licht codieren, von dem optischen Lesegerät (42) zu empfangen und eine Authentifizierungsaktion an dem Muster von emittiertem Licht durchzuführen.

11. Authentifizierungsassay (100) unter Verwendung eingebetteter Desoxyribonukleinsäure-, DNA-, Marker, wobei das Assay Folgendes umfasst:
ein Substrat (10) mit einer Vielzahl von physikalisch getrennten Assaystellen (12), wobei jede Assaystelle (12) der Vielzahl von Assaystellen (12) ein Reporter-Oligonukleotid (14) aufweist, das über eine Ankerregion (18) an das Substrat (10) gebunden ist; und
eine Probe einer Authentizitätsmarkierung, die von einem Produkt gesammelt wurde, wobei
jedes Reporter-Oligonukleotid (14), das an jede Assaystelle (12) der Vielzahl von Assaystellen (12) gebunden ist, eine erste Region (21) und eine zweite Region (22) aufweist,
die erste Region (21) jedes Reporter-Oligonukleotids (14) eine universelle Sequenz aufweist, die unter jedem der Reporter-Oligonukleotide (14), die an jede Assaystelle (12) der Vielzahl von Assaystellen (12) gebunden sind, gemeinsam ist,
die zweite Region (22) jedes Reporter-Oligonukleotids (14) eine eindeutige Sequenz aufweist, die für jedes der Reporter-Oligonukleotide (14), die an jede Assaystelle (12) der Vielzahl von Assaystellen (12) gebunden sind, unterschiedlich ist,
die erste und zweite Region (21, 22) jedes Reporter-Oligonukleotids (14) mit einem komplementären Strang (20) vorhybridisiert sind, der eine Sequenz aufweist, die zu der ersten und zweiten Region (21, 22) jedes Reporter-Oligonukleotids (14) komplementär ist, wobei der komplementäre Strang (20) eine Toehold-Sequenz und ein Fluorophormolekül aufweist,
die Probe mindestens einen DNA-Marker (26) aufweist, der eine gleiche Sequenz wie die erste und zweite Region (21, 22) mindestens eines Reporter-Oligonukleotids (14), das an eine Assaystelle (12) der Vielzahl von Assaystellen (12) gebunden ist, und eine Toehold-Sequenz aufweist, die zu der Toehold-Sequenz des komplementären Strangs (20) komplementär ist,
eine Inkubation des Substrats (10) mit der Probe zu einer Bindung des mindestens einen DNA-Markers (26) an den komplementären Strang (20) führt, wodurch eine Toehold-vermittelte DNA-Strang (20)-Verdrängungsreaktion initiiert wird, die den komplementären Strang (20), der das Fluorophormolekül aufweist, von dem mindestens einen Reporter-Oligonukleotid (14) verdrängt, und
eine Exposition des Substrats (10) gegenüber Licht, das dazu konfiguriert ist, Fluorophormoleküle anzuregen, ein Muster von emittiertem Licht an einer oder mehreren Assaystellen (12) erzeugt, wobei das Muster eine Abwesenheit von emittiertem Licht an einer oder mehreren Assaystellen (12) der Vielzahl von Assaystellen (12) aufgrund einer Freigabe des komplementären Strangs (20), der das Fluorophormolekül aufweist, aufweist.

12. Authentifizierungsassay (100) nach Anspruch 11, wobei
der mindestens eine DNA-Marker (26) einer einer Vielzahl von DNA-Markern (26) ist, die in der Probe enthalten sind, und
jeder DNA-Marker (26) der Vielzahl von DNA-Markern (26), die in der Probe enthalten sind, eine zweite Region (22) mit einer gleichen eindeutigen Sequenz wie eine zweite Region (22) eines Reporter-Oligonukleotids (14), das an eine der Assaystellen (12) der Vielzahl von Assaystellen (12) gebunden ist, aufweist.

13. Authentifizierungsassay (100) nach Anspruch 11, wobei
die Probe zusätzlich mindestens einen Decoy-DNA-Marker (26) aufweist, der eine gleiche universelle Sequenz wie die erste Region (21) mindestens eines Reporter-Oligonukleotids (14), eine andere Sequenz als eine eindeutige Sequenz einer zweiten Region (22) des mindestens einen Reporter-Oligonukleotids (14) und eine Toehold-Sequenz, die zu der Toehold-Sequenz des komplementären Strangs (20) komplementär ist, aufweist, und
der DNA-Marker (26) mit einem blockierenden Strang (20) vorhybridisiert ist, der eine gleiche Sequenz wie die Toehold-Sequenz und die erste Region (21) des komplementären Strangs (20) aufweist, wodurch die Toehold-vermittelte DNA-Strang (20)-Verdrängungsreaktion blockiert wird, die den komplementären Strang (20), der das Fluorophormolekül aufweist, von dem mindestens einen Reporter-Oligonukleotid (14) verdrängt.

14. System (102), das zum Analysieren des Authentifizierungsassays (100) nach Anspruch 11 konfiguriert ist, umfassend:
eine lichtemittierende Vorrichtung, die dazu konfiguriert ist, Licht mit einer Wellenlänge zu emittieren, die eine Anregung eines Fluorophormoleküls, das an den komplementären Strang (20) gebunden ist, bewirkt;
ein optisches Lesegerät (42), das dazu konfiguriert ist, das Muster von emittiertem Licht, einschließlich der Abwesenheit von emittiertem Licht, an einer oder mehreren Assaystellen (12) zu erfassen; und
eine Rechenvorrichtung, die dazu konfiguriert ist, Daten, die das Muster von emittiertem Licht codieren, von dem optischen Lesegerät (42) zu empfangen, eine Verifizierungsaktion an dem Muster von emittiertem Licht durchzuführen und ein Verifizierungsergebnis auszugeben, wobei
die lichtemittierende Vorrichtung ein Handlicht ist,
das optische Lesegerät (42) eine mit einer Kamera ausgestattete mobile Rechenvorrichtung ist, und
die Rechenvorrichtung ein Server ist, der dazu konfiguriert ist, die Daten, die das Muster von emittiertem Licht codieren, über ein Computernetzwerk von der mobilen Rechenvorrichtung zu empfangen.

15. Authentifizierungsassay (100) nach Anspruch 11, wobei
die Authentizitätsmarkierung in einer Beschichtung enthalten ist, die auf eine Oberfläche des Produkts aufgebracht ist.

## Revendications

1. Dosage d'authentification (100) utilisant des traceurs d'acide désoxyribonucléique, ADN, incorporés, le dosage comprenant :
un substrat (10) ayant une pluralité d'emplacements de dosage (12) physiquement séparés, chaque emplacement de dosage (12) de la pluralité d'emplacements de dosage (12) comportant un oligonucléotide rapporteur (14) lié au substrat (10) via une région d'ancrage (18) ; et
un échantillon d'un marqueur d'authenticité prélevé sur un produit, dans lequel
chaque oligonucléotide rapporteur (14) lié à chaque emplacement de dosage (12) de la pluralité d'emplacements de dosage (12) comporte une première région (21), une deuxième région (22) et une troisième région (23),
la première région (21) de chaque oligonucléotide rapporteur (14) comporte une séquence d'accrochage (20) simple brin qui est commune à chacun des oligonucléotides rapporteurs (14) liés à chaque emplacement de dosage (12) de la pluralité d'emplacements de dosage (12),
la deuxième région (22) de chaque oligonucléotide rapporteur (14) comporte une séquence universelle qui est commune à chacun des oligonucléotides rapporteurs (14) liés à chaque emplacement de dosage (12) de la pluralité d'emplacements de dosage (12),
la troisième région (23) de chaque oligonucléotide rapporteur (14) comporte une séquence unique qui est différente pour chacun des oligonucléotides rapporteurs (14) liés à chaque emplacement de dosage (12) de la pluralité d'emplacements de dosage (12),
les deuxième et troisième régions (22, 23) de chaque oligonucléotide rapporteur (14) sont préhybridées avec un brin complémentaire (20) ayant une séquence qui est complémentaire aux deuxième et troisième régions (22, 23) de chaque oligonucléotide rapporteur (14),
l'échantillon comporte au moins un traceur d'ADN (26) ayant une séquence complémentaire aux première et deuxième régions (21, 22) de chacun des oligonucléotides rapporteurs (14), et complémentaire à la troisième région (23) d'au moins un oligonucléotide rapporteur (14) lié à un emplacement de dosage (12) de la pluralité d'emplacements de dosage (12), l'au moins un traceur d'ADN (26) comportant une molécule de fluorophore configurée pour émettre une lumière lors de son excitation,
l'incubation du substrat (10) avec l'échantillon entraîne la liaison de l'au moins un traceur d'ADN (26) à l'au moins un oligonucléotide rapporteur (14), ce qui initie une réaction de déplacement de brin d'ADN (20) médiée par accrochage qui échange le brin complémentaire (20) de l'au moins un oligonucléotide rapporteur (14) contre l'au moins un traceur d'ADN (26) comportant la molécule de fluorophore, et
l'excitation de la molécule de fluorophore fixée au traceur d'ADN (26) amène la molécule de fluorophore à émettre une lumière, ce qui donne un motif de lumière émise à un ou plusieurs emplacements de dosage (12).

2. Dosage d'authentification (100) selon la revendication 1, dans lequel
l'au moins un traceur d'ADN (26) est l'un d'une pluralité de traceurs d'ADN (26) inclus dans l'échantillon, et
chaque traceur d'ADN (26) de la pluralité de traceurs d'ADN (26) inclus dans l'échantillon présente une troisième région (23) avec une séquence unique complémentaire à une troisième région (23) d'un oligonucléotide rapporteur (14) lié à l'un des emplacements de dosage (12) de la pluralité d'emplacements de dosage (12).

3. Dosage d'authentification (100) selon la revendication 1, dans lequel
l'échantillon comporte en outre au moins un traceur d'ADN leurre (26) ayant une troisième région (23) avec une séquence unique complémentaire à un oligonucléotide rapporteur (14) lié à l'un des emplacements de dosage (12) de la pluralité d'emplacements de dosage (12), l'au moins un traceur d'ADN leurre (26) étant dépourvu de molécule de fluorophore.

4. Système (102) configuré pour analyser le dosage d'authentification (100) selon la revendication 1, comprenant :
un dispositif émetteur de lumière configuré pour émettre une lumière à une longueur d'onde qui provoque l'excitation de la molécule de fluorophore fixée au traceur d'ADN (26) ;
un lecteur optique (42) configuré pour capturer le motif de lumière émise ; et
un dispositif informatique configuré pour recevoir des données codant le motif de lumière émise provenant du lecteur optique (42), effectuer une action de vérification sur le motif de lumière émise, et délivrer un résultat de vérification.

5. Système (102) selon la revendication 4, dans lequel
le dispositif émetteur de lumière est une lampe portative,
le lecteur optique (42) est un dispositif informatique mobile équipé de caméra, et
le dispositif informatique est un serveur configuré pour recevoir les données codant le motif de lumière émise sur un réseau informatique à partir du dispositif informatique mobile.

6. Méthode de fabrication d'un dosage d'authentification (100) utilisant des traceurs d'acide désoxyribonucléique, ADN, incorporés, la méthode comprenant :
la préparation (302) d'un substrat (10) pour avoir une pluralité d'emplacements de dosage (12) physiquement séparés ;
la synthèse (304) d'une pluralité d'oligonucléotides rapporteurs (14), chacun présentant une première région (21) comportant une séquence d'accrochage (20) simple brin qui est commune à chacun des oligonucléotides rapporteurs (14), une deuxième région (22) comportant une séquence universelle qui est commune à chacun des oligonucléotides rapporteurs (14), et une troisième région (23) comportant une séquence unique qui est différente pour chacun des oligonucléotides rapporteurs (14) ;
la préhybridation (306) des deuxième et troisième régions (22, 23) de chaque oligonucléotide rapporteur (14) de la pluralité d'oligonucléotides rapporteurs (14) avec un brin complémentaire (20) ayant une séquence qui est complémentaire aux deuxième et troisième régions (22, 23) de chaque oligonucléotide rapporteur (14) ;
la liaison (308), via une région d'ancrage (18), de chaque oligonucléotide rapporteur (14) de la pluralité d'oligonucléotides rapporteurs (14) à l'un de la pluralité d'emplacements de dosage (12) physiquement séparés pour que chaque emplacement de dosage (12) comporte un oligonucléotide rapporteur (14) avec une séquence unique dans la troisième région (23) ;
la synthèse (310) d'au moins un traceur d'ADN (26) pour avoir une séquence complémentaire aux première et deuxième régions (21, 22) de chacun des oligonucléotides rapporteurs (14), et complémentaire à la troisième région (23) d'au moins un oligonucléotide rapporteur (14) lié à un emplacement de dosage (12) de la pluralité d'emplacements de dosage (12), l'au moins un traceur d'ADN (26) comportant une molécule de fluorophore configurée pour émettre une lumière lors de son excitation ; et
l'ajout (312) de l'au moins un traceur d'ADN (26) à un marqueur d'authenticité configuré pour vérifier une authenticité d'un produit, dans lequel
l'incubation du substrat (10) avec un échantillon du marqueur d'authenticité entraîne la liaison de l'au moins un traceur d'ADN (26) à l'au moins un oligonucléotide rapporteur (14), ce qui initie une réaction de déplacement de brin d'ADN (20) médiée par accrochage qui échange le brin complémentaire (20) de l'au moins un oligonucléotide rapporteur (14) contre l'au moins un traceur d'ADN (26) comportant la molécule de fluorophore, et
l'excitation de la molécule de fluorophore fixée au traceur d'ADN (26) amène la molécule de fluorophore à émettre une lumière, ce qui donne un motif de lumière émise à un ou plusieurs emplacements de dosage (12).

7. Méthode selon la revendication 6, dans laquelle l'au moins un traceur d'ADN (26) est l'un d'une pluralité de traceurs d'ADN (26) inclus dans l'échantillon, et
la méthode comprend en outre la synthèse de chaque traceur d'ADN (26) de la pluralité de traceurs d'ADN (26) inclus dans l'échantillon pour avoir une troisième région (23) avec une séquence unique complémentaire à un oligonucléotide rapporteur (14) lié à l'un des emplacements de dosage (12) de la pluralité d'emplacements de dosage (12).

8. Méthode selon la revendication 6, la méthode comprenant en outre :
la synthèse d'au moins un traceur d'ADN leurre (26) ayant une troisième région (23) avec une séquence unique complémentaire à un oligonucléotide rapporteur (14) lié à l'un des emplacements de dosage (12) de la pluralité d'emplacements de dosage (12), l'au moins un traceur d'ADN leurre (26) étant dépourvu de molécule de fluorophore ; et
comportant l'au moins un traceur d'ADN leurre (26) dans le marqueur d'authenticité.

9. Méthode selon la revendication 6, la méthode comprenant en outre :
l'excitation de la molécule de fluorophore fixée au traceur d'ADN (26) avec un dispositif émetteur de lumière ; et
la capture du motif de lumière émise avec un lecteur optique (42).

10. Méthode selon la revendication 9, la méthode comprenant en outre :
la réception d'un résultat d'authentification délivré par un dispositif informatique configuré pour recevoir des données codant le motif de lumière émise provenant du lecteur optique (42) et effectuer une action d'authentification sur le motif de lumière émise.

11. Dosage d'authentification (100) utilisant des traceurs d'acide désoxyribonucléique, ADN, incorporés, le dosage comprenant :
un substrat (10) ayant une pluralité d'emplacements de dosage (12) physiquement séparés, chaque emplacement de dosage (12) de la pluralité d'emplacements de dosage (12) comportant un oligonucléotide rapporteur (14) lié au substrat (10) via une région d'ancrage (18) ; et
un échantillon d'un marqueur d'authenticité prélevé sur un produit, dans lequel
chaque oligonucléotide rapporteur (14) lié à chaque emplacement de dosage (12) de la pluralité d'emplacements de dosage (12) comporte une première région (21) et une deuxième région (22),
la première région (21) de chaque oligonucléotide rapporteur (14) comporte une séquence universelle qui est commune à chacun des oligonucléotides rapporteurs (14) liés à chaque emplacement de dosage (12) de la pluralité d'emplacements de dosage (12),
la deuxième région (22) de chaque oligonucléotide rapporteur (14) comporte une séquence unique qui est différente pour chacun des oligonucléotides rapporteurs (14) liés à chaque emplacement de dosage (12) de la pluralité d'emplacements de dosage (12),
les première et deuxième régions (21, 22) de chaque oligonucléotide rapporteur (14) sont préhybridées avec un brin complémentaire (20) ayant une séquence qui est complémentaire aux première et deuxième régions (21, 22) de chaque oligonucléotide rapporteur (14), le brin complémentaire (20) comportant une séquence d'accrochage et une molécule de fluorophore,
l'échantillon comporte au moins un traceur d'ADN (26) ayant une séquence identique à celles des première et deuxième régions (21, 22) d'au moins un oligonucléotide rapporteur (14) lié à un emplacement de dosage (12) de la pluralité d'emplacements de dosage (12), et une séquence d'accrochage complémentaire à la séquence d'accrochage du brin complémentaire (20),
l'incubation du substrat (10) avec l'échantillon entraîne la liaison de l'au moins un traceur d'ADN (26) au brin d'ADN complémentaire (20), ce qui initie une réaction de déplacement de brin d'ADN (20) médiée par accrochage qui déplace le brin d'ADN complémentaire (20), comportant la molécule de fluorophore, à partir de l'au moins un oligonucléotide rapporteur (14), et
l'exposition du substrat (10) à une lumière configurée pour exciter des molécules de fluorophore produit un motif de lumière émise à un ou plusieurs emplacements de dosage (12), le motif comportant une absence de lumière émise à un ou plusieurs emplacements de dosage (12) de la pluralité d'emplacements de dosage (12) en raison de la libération du brin complémentaire (20), comportant la molécule de fluorophore.

12. Dosage d'authentification (100) selon la revendication 11, dans lequel
l'au moins un traceur d'ADN (26) est l'un d'une pluralité de traceurs d'ADN (26) inclus dans l'échantillon, et
chaque traceur d'ADN (26) de la pluralité de traceurs d'ADN (26) inclus dans l'échantillon présente une deuxième région (22) avec une séquence unique identique à celle d'une deuxième région (22) d'un oligonucléotide rapporteur (14) lié à l'un des emplacements de dosage (12) de la pluralité d'emplacements de dosage (12).

13. Dosage d'authentification (100) selon la revendication 11, dans lequel
l'échantillon comporte en outre au moins un traceur d'ADN leurre (26) ayant une séquence universelle identique à celle de la première région (21) d'au moins un oligonucléotide rapporteur (14), une séquence différente d'une séquence unique d'une deuxième région (22) de l'au moins un oligonucléotide rapporteur (14), et une séquence d'accrochage complémentaire à la séquence d'accrochage du brin complémentaire (20), et
le traceur d'ADN (26) est préhybridé avec un brin de blocage (20) ayant une séquence identique à celle de la séquence d'accrochage et une première région (21) du brin complémentaire (20), ce qui bloque la réaction de déplacement de brin d'ADN (20) médiée par accrochage qui déplace le brin complémentaire (20), comportant la molécule de fluorophore, de l'au moins un oligonucléotide rapporteur (14).

14. Système (102) configuré pour analyser le dosage d'authentification (100) selon la revendication 11, comprenant :
un dispositif émetteur de lumière configuré pour émettre une lumière à une longueur d'onde qui provoque l'excitation d'une molécule de fluorophore fixée au brin complémentaire (20) ;
un lecteur optique (42) configuré pour capturer le motif de lumière émise, y compris l'absence de lumière émise à un ou plusieurs emplacements de dosage (12) ; et
un dispositif informatique configuré pour recevoir des données codant le motif de lumière émise provenant du lecteur optique (42), effectuer une action de vérification sur le motif de lumière émise, et délivrer un résultat de vérification, dans lequel
le dispositif émetteur de lumière est une lampe portative,
le lecteur optique (42) est un dispositif informatique mobile équipé de caméra, et
le dispositif informatique est un serveur configuré pour recevoir les données codant le motif de lumière émise sur un réseau informatique à partir du dispositif informatique mobile.

15. Dosage d'authentification (100) selon la revendication 11, dans lequel
le marqueur d'authenticité est inclus dans un enrobage appliqué sur une surface du produit.
